# EUROPÄISCHE PATENTSCHRIFT

(11) **EP 2 079 714 B1**
(45) Veröffentlichungstag und Bekanntmachung des Hinweises auf die Patenterteilung: **26.09.2012**
(21) Anmeldenummer: 07821247.9
(22) Anmeldetag: 12.10.2007
(51) Int. Cl.: A01N 43/54, A01N 43/48, C07D 239/54

(54) **HYDRATE DES 2-CHLOR-5-Ý3,6-DIHYDRO-3-METHYL-2,6-DIOXO-4-(TRIFLUORMETHYL)-1-(2H)-PYRIMIDINYL¨-4-FLUOR-N-ÝÝMETHYL-(1-METHYLETHYL)AMINO¨SULFONYL¨BENZAMIDS**
HYDRATES OF 2-CHLORO-5-Ý3,6-DIHYDRO-3-METHYL-2,6-DIOXO-4-(TRIFLUORO-METHYL)-1-(2H)-PYRIMIDINYL¨-4-FLUORO-N-ÝÝMETHYL-(1-METHYLETHYL)-AMINO¨SULPHONYL¨BENZAMIDE
HYDRATES DE 2-CHLORO-5-Ý3,6-DIHYDRO-3-MÉTHYL-2,6-DIOXO-4-(TRIFLUORO-MÉTHYL)-1-(2H)-PYRIMIDINYL¨-4-FLUORO-N-ÝÝMÉTHYL-(1-MÉTHYLÉTHYL)AMINO¨-SULFONYL¨BENZAMIDE

(30) Priorität: 13.10.2006 EP 06122264
(43) Veröffentlichungstag der Anmeldung: 22.07.2009
(73) Patentinhaber: BASF SE, 67056 Ludwigshafen (DE)
(72) Erfinder: SCHMIDT, Thomas, 67433 Neustadt (DE); GEBHARDT, Joachim, 67157 Wachenheim (DE); LÖHR, Sandra, 67059 Ludwigshafen (DE); KEIL, Michael, 67251 Freinsheim (DE); WEVERS, Jan Hendrik, 67591 Hohensülzen (DE); ERK, Peter, 67227 Frankenthal (DE); SAXELL, Heidi Emilia, 67071 Ludwigshafen (DE); HAMPRECHT, Gerhard, 69469 Weinheim (DE); SEITZ, Werner, 68723 Plankstadt (DE); MAYER, Guido, 67161 Gönnheim (DE); WOLF, Bernd, 67136 Fussgönheim (DE); COX, Gerhard, 67098 Bad Dürkheim (DE); MICHEL, Alfred, 67305 Ramsen (DE); ZAGAR, Cyrill, Kowloon,Hong Kong (CN); REINHARD, Robert, 67117 Limburgerhof (DE); SIEVERNICH, Bernd, 67454 Hassloch (DE)
(74) Vertreter: Reitstötter - Kinzebach
(86) Internationale Anmeldenummer: PCT/EP2007/060880
(87) Internationale Veröffentlichungsnummer: WO 2008/043836

(56) Entgegenhaltungen:
- WO-A-01/83459
- WO-A-03/097589
- WO-A-2005/054208
- WO-A-2006/010474

## Beschreibung

Die vorliegende Erfindung betrifft kristalline Hydrate des 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]-sulfonyl]benzamids, das im Folgenden auch als Phenyluracil I bezeichnet wird. Die Erfindung betrifft auch ein Verfahren zur Herstellung dieser Hydrate sowie Formulierungen für den Pflanzenschutz, welche Hydrate des Phenyluracils I enthalten.

Bei dem Phenyluracil I, welches der folgenden Formel entspricht, 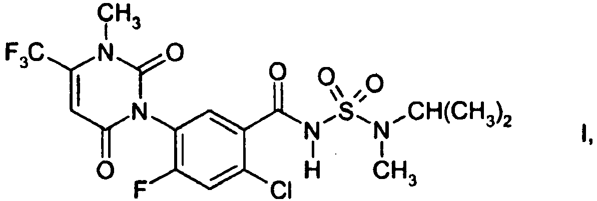 handelt es sich um einen herbiziden Wirkstoff, welcher aus der WO 01/083459 bekannt ist. Weitere Verfahren zu seiner Herstellung sind aus WO 03/097589, WO 05/054208 und WO 06/097589 sowie aus der älteren internationalen Anmeldung PCT/EP 2006/062414 bekannt. Alle bekannten Verfahren zur Herstellung des Phenyluracils I, liefern dieses als eine amorphe Substanz.

Eigene Untersuchungen der Anmelderin haben gezeigt, dass das amorphe Phenyluracil I nur bedingt zur Herstellung von Formulierungen, welche die Substanz als Feststoff enthalten, geeignet ist. Insbesondere bei mehrphasigen Formulierungen können Stabilitätsprobleme auftreten.

Es wurde nunmehr überraschenderweise gefunden, dass durch geeignete Verfahren kristalline Hydrate des Phenyluracils I erhalten werden, welche diese Nachteile nicht aufweisen. Zudem hat sich überraschenderweise gezeigt, dass diese Hydrate eine bessere herbizide Wirksamkeit und in einer Reihe von Kulturen eine bessere Nutzpflanzenverträglichkeit aufweisen als die bislang bekannte amorphe Form des Phenyluracils I. Bei den Hydraten des Phenyluracils I handelt es sich um kristalline Substanzen, die kompakter als die bislang bekannte amorphe Form sind. Die Hydrate sind daher leichter zu handhaben als die bislang bekannte amorphe Form des Phenyluracils I.

Dementsprechend betrifft die vorliegende Erfindung kristalline Hydrate des 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamids.

Bei den Hydraten des Phenyluracils I handelt es sich um kristalline Substanzen die, abhängig von der Ausbildung der Kristallite, etwa 0,8 bis 1,2 Mol Wasser, insbesondere 0,9 bis 1,1 Mol und speziell 0,95 bis 1,05 Mol pro Mol Phenyluracils enthalten und die daher als Monohydrate aufzufassen sind. Die Zusammensetzung lässt sich durch eine Wasserbestimmung nach Karl-Fischer bestimmen.

Die Hydrate schmelzen, abhängig von der Aufheizrate und der Art der Probengefäße, in einem Temperaturbereich von 100 bis 150 °C. In offenen Probegefäßen und bei niedrigen Aufheizraten von bis zu 2 K/min schmelzen die Hydrate in einem Bereich von 100 bis 130 °C, wohingegen der Schmelzbereich bei höheren Aufheizraten und/oder bei Verwendung geschlossener Probengefäße zu höheren Werten verschoben wird. In verschlossenen Probengefäßen und Aufheizraten von 5 K/min liegt der Schmelzbereich typischerweise im Bereich von 120 bis 150 °C, mit einem Peak-Maximum im Bereich von 130 bis 140 °C, Die hier angegebenen Schmelzpunkte beziehen sich auf mittels Differentialkalorimetrie (Differential Scanning Calorimetry: DSC, Tiegelmaterial Aluminium) ermittelte Werte.

Eine genauere Untersuchung der Hydrate hat ergeben, dass diese in zwei unterschiedlichen Formen, im Folgenden auch als Hydrat (a) und Hydrat (b) bezeichnet, anfallen können, die jedoch eine identische Zusammensetzung und vergleichbare Schmelzpunkte aufweisen. Zur Unterscheidung von der bekannten amorphen Form, im Folgenden auch als Form I bezeichnet, wird das erfindungsgemäße Hydrat (a) im Folgenden auch als Form III und das erfindungsgemäße Hydrat (b) im Folgenden auch als Form IV bezeichnet.

Die erfindungsgemäße Form III kann mittels Röntgenpulverdiffraktometrie anhand ihres Beugungsdiagramms identifiziert werden. So zeigt ein bei 25 °C unter Verwendung von Cu-K_{α}-Strahlung (1,54178 Å) aufgenommenes Röntgenpulverdiffraktogramm der Form III zumindest einen charakteristischen Reflex bei 2θ = 11,6 ± 0,2°, wohingegen die Form IV zumindest einen charakteristischen Reflex bei 2θ = 12,1 ± 0,2° zeigt.

Insbesondere zeigt ein Röntgenpulverdiffraktogramm der Form III unter diesen Bedingungen zusätzlich zu dem Reflex bei 2θ = 11,6 ± 0,2° wenigstens 3, häufig wenigstens 5, insbesondere wenigstens 7 weitere und speziell alle der in der folgenden Tabelle 1 als 2θ-Werte, bzw. als Netzebenenabstände d, angegebenen Reflexe:

**Tabelle 1:**

| 2θ | d [Å] |
|---|---|
| 5,1 ± 0.2° | 17,37 ± 0,02 |
| 10,1 ± 0,2° | 8,76 ± 0,02 |
| 10,8 ± 0,2° | 8,15 ± 0,02 |
| 11,6 ± 0,2° | 7,62 ± 0,02 |
| 13,9 ± 0,2° | 6,35 ± 0,02 |
| 15,1 ± 0,2° | 5,86 ± 0,02 |
| 16,1 ± 0,2° | 5,49 ± 0,02 |
| 17,9 ± 0,2° | 4,95 ± 0,02 |
| 20,2 ± 0,2° | 4,40 ± 0,02 |
| 24,5 ± 0,2° | 3,63 ± 0,02 |

Untersuchungen an Einkristallen der Form III zeigen, dass die zugrunde liegende Kristallstruktur monoklin ist. Die Elementarzelle weist die Raumgruppe P2(1)/c auf. Die charakteristischen Daten der Kristallstruktur von Form III (bestimmt bei -170°C) sind in der Tabelle 2 zusammengefasst.

**Tabelle 2: Kristallographische Eigenschaften der Form III**

| Parameter | Form III |
|---|---|
| Klasse | monoklin |
| Raumgruppe | P2(1)/c |
| a | 17,624(2) Å |
| b | 9,012(1) Å |
| c | 13,624(1)Å |
| α | 90° |
| β | 92,223(4)° |
| γ | 90° |
| Volumen | 2162,3(3) Å ³ |
| Z | 4 |
| Dichte (berechnet) | 1,594 Mg/m³ |
| R¹ ; wR² | 0,096; 0,278 |
| Wellenlänge | 1,54178 Å |

| | |
|---|---|
| a,b,c = Kantenlänge der Elementarzelle α,β,γ = Winkel der Elementarzelle Z = Anzahl der Moleküle in der Elementarzelle | |

Ein bei 25 °C unter Verwendung von Cu-K_{α}-Strahlung (1,54178 Å) aufgenommenes Röntgenpulverdiffraktogramm der Form IV wiederum zeigt zusätzlich zu dem Reflex bei 2θ = 12,03 ± 0,2° in der Regel wenigstens 3, häufig wenigstens 5, insbesondere wenigstens 7 weitere und speziell alle der in der folgenden Tabelle 3 als 2θ-Werte, bzw. als Netzebenenabstände d, angegebenen Reflexe:

**Tabelle 3:**

| 2θ | d[Å] |
|---|---|
| 5,2 ± 0,2° | 17,21 ± 0,02 |
| 10,2 ± 0,2° | 8,63 ± 0,02 |
| 10,9 ± 0,2° | 8,11 ± 0,02 |
| 12,1 ± 0,2° | 7,32 ± 0,02 |
| 14.0 ± 0.2° | 6,31 ± 0,02 |
| 14,6 ± 0,2° | 6,08 ± 0,02 |
| 15,4 ± 0,2° | 5,77 ± 0,02 |
| 19,2 ± 0,2° | 4,61 ± 0,02 |
| 19,9 ± 0,2° | 4,44 ± 0,02 |
| 20.5 ± 0,2° | 4,33 ± 0,02 |
| 24,7 ± 0,2° | 3,60 ± 0,02 |
| 26,7 ± 0,2° | 3,34 ± 0,02 |
| 27,8 ± 0,2° | 3,21 ± 0,02 |

Die Herstellung der Hydrate des Phenyluracils I gelingt beispielsweise durch die im Folgenden beschriebenen Verfahren, welche als zentralen Schritt eine Kristallisation aus einer Lösung des Phenyluracils I in einem organischen Lösungsmittel in Gegenwart von Wasser umfassen.

Bei den organischen Lösungsmitteln kann es sich sowohl um solche organischen Lösungsmittel handeln, die mit Wasser mischbar sind, als auch um solche, die nur eine begrenzte Mischbarkeit mit Wasser aufweisen.

Beispiele für bevorzuge organische Lösungsmittel, die im Folgenden auch als Lösungsmittel L1 bezeichnet werden, sind
- acyclische Ether mit 4 bis 6 C-Atomen wie Methyl-tert.-butylether,
- alicyclische Ether mit 4 bis 6 C-Atomen wie Tetrahydrofuran,
- Dialkylketone mit 3 bis 5 C-Atomen, insbesondere Aceton,
- C₁-C₄-Alkanole wie Methanol, Ethanol, n-Propanol, Isopropanol, tert.-Butanol,
- C₂C₃-Alkylenglykol-mono-C₁-C₄-alkylether wie Ethylenglykolmethylether und Ethylenglykol-n-propylether,
- Di-(C₂C₃-alkylenglykol-mono-C₁-C₄-alkylether,
- C₁-C₄-Alkylester aliphatischer C₁-C₄-Carbonsäuren, insbesondere C₁-C₄-Alkylester der Essigsäure wie Ethylacetat und Butylacetat,
- aliphatische und aromatische Kohlenwasserstoffe, insbesondere Mono- und Di-C₁-C₄-Alkylbenzole wie Toluol und Xylol sowie C₅-C₈-Alkane wie Pentan, Heptan und Hexan,
- aliphatische und aromatische Chlorkohlenwasserstoffe wie Dichlormethan und Chlorbenzol, und
- Mischungen dieser Lösungsmittel.

Besonders bevorzugte organische Lösungsmittel sind Methanol sowie Mono- und Di-C₁-C₄-Alkylbenzole, insbesondere Toluol, sowie Gemische von Mono- und Di-C₁-C₄-Alkylbenzolen, insbesondere Toluol, mit den vorgenannten Lösungsmitteln L1, insbesondere mit Tetrahydrofuran und/oder mit Methanol. In diesen Mischungen macht das Mono- bzw. Di-C₁-C₄-Alkylbenzol wenigstens 50 Vol.-% und insbesondere wenigstens 80 Vol.-%, bezogen auf die Gesamtmenge an organischem Lösungsmittel L1, aus.

Neben den vorgenannten Lösungsmitteln L1 kann das zur Kristallisation eingesetzte organische Lösungsmittel auch andere, davon verschiedene organische Lösungsmittel (im folgenden Lösungsmittel L2) enthalten. Der Anteil der Lösungsmittel L2 beträgt in der Regel nicht mehr als 50 Vol.-%, insbesondere nicht mehr als 20 Vol.-%, bezogen auf die Gesamtmenge an dem zur Kristallisation eingesetzten organischem Lösungsmittel.

Die Kristallisation der Hydrate wird erfindungsgemäß in Gegenwart von Wasser durchgeführt. Dieses wird in der Regel einer Lösung des Phenyluracils in dem organischen Lösungsmittel zugesetzt. Im Falle von Lösungsmitteln, die Wasser zumindest teilweise zu lösen vermögen, wird durch den Wasserzusatz die Löslichkeit des Phenyluracils in dem organischen Lösungsmittel erniedrigt und eine Kristallisation des Hydrats tritt ein. Im Falle von Lösungsmitteln, die mit der zugesetzten Wassermenge ein mehrphasiges System bilden, wird die Kristallisation in der Regel durch Abkühlen bewirkt.

Die Menge an Wasser liegt typischerweise im Bereich von 5 bis 300 Volumenteilen, insbesondere im Bereich von 5 bis 200 Volumenteilen, bezogen auf 100 Volumenteile organisches Lösungsmittel.

Häufig wird man zur Kristallisation der Hydrate so vorgehen, dass man eine Lösung des Phenyluracils I in einem der vorgenannten organischen Lösungsmittel bereitstellt und hierzu die gewünschte Wassermenge zugibt.

Die Zugabe des Wassers kann bei Raumtemperatur oder bei erhöhter Temperatur erfolgen und erfolgt typischerweise bei Temperaturen von 20 °C bis hin zur Siedetemperatur des eingesetzten organischen Lösungsmittels, vorzugsweise jedoch bei Temperaturen bis maximal 90 °C. Die gewünschte Wassermenge kann in einer oder mehreren Portionen über einen kurzen Zeitraum, d. h. bis 10 min, oder langsam, z. B. über einen Zeitraum von wenigstens 30 min oder wenigstens 60 min, z. B. 30 bis 300 min oder 60 bis 300 min oder 120 bis 300 min erfolgen.

Sofern die Zugabe des Wassers bei erhöhter Temperatur erfolgt, wird man in der Regel im Anschluss an die Zugabe des Wassers die Temperatur erniedrigen. Beispielsweise kann man so vorgehen, dass man zu der heißen Lösung Wasser gibt, bis die Kristallisation beginnt, anschließend abkühlt und gegebenenfalls weiteres Wasser zur Vervollständigung der Kristallisation zugibt.

Es ist ebenfalls möglich, das Phenyluracil I, vorzugsweise die amorphe Form, in einem geeigneten Gemisch aus organischem Lösungsmittel und Wasser, gegebenenfalls unter Erwärmen, z. B. auf Temperaturen im Bereich von 40 bis 90 °C, zu lösen und die Kristallisation durch Zusatz von weiterem Wasser und/oder durch Abkühlen zu bewirken.

Zur Kristallisation der Form III sind beispielsweise solche Verfahren geeignet, die eine langsame Kristallisation bewirken, z. B. indem man die Zugabe des Wassers über einen längeren Zeitraum durchführt und/oder die das Phenyluracil gelöst enthaltene Mischung aus Lösungsmittel und Wasser langsam abkühlt.

Die Bildung der Form IV kann wiederum durch solche Maßnahmen gefördert werden, die eine rasche Kristallisation bewirken, z. B. eine rasche Zugabe von Wasser oder die Durchführung der Kristallisation bei Temperaturen unterhalb 40 °C.

Die Kristallisation der Form III oder IV lässt sich durch Animpfen mit Impfkristallen der jeweiligen Form steuern oder beschleunigen, beispielsweise indem vor oder während der Kristallisation Impfkristalle der jeweiligen Form zugesetzt werden.

Sofern man bei der Kristallisation Impfkristalle zusetzt, beträgt ihre Menge typischerweise 0,001 bis 10 Gew.-%, häufig 0,005 bis 5 Gew.-%, insbesondere 0,01 bis 1 Gew.-% und speziell 0,05 bis 0,5 Gew.-%, bezogen auf das gelöste Phenyluracil I.

Sofern man die Kristallisation in Gegenwart von Impfkristallen durchführt, werden diese vorzugsweise erst bei einer Temperatur zugegeben, bei der die Sättigungskonzentration des Phenyluracils I in dem jeweiligen Lösungsmittel erreicht ist, d.h. bei oder unterhalb derjenigen Temperatur, bei der die gelöste Menge an Phenyluracil I in dem jeweiligen Lösungsmittel eine gesättigte Lösung bildet. Die Temperaturabhängigkeit der Sättigungskonzentration in einem Lösungsmittel kann der Fachmann in Routineexperimenten ermitteln. Häufig erfolgt die Zugabe der Impfkristalle, wenn die Temperatur der Lösung nicht mehr als 60 °C beträgt. Vorzugsweise lässt man nach der Zugabe der Impfkristalle den Ansatz auf Temperaturen unterhalb 30 °C, insbesondere von 25 °C oder darunter, z. B. auf Temperaturen im Bereich von 0 °C bis 25 °C abkühlen, bevor man zur Isolierung der Hydrate des Phenyluracils I das erhaltene kristalline Material von der Mutterlauge abtrennt. Das Abkühlen bei Anwesenheit von lmpfkristallen kann kontrolliert mit einer Abkühlrate von in der Regel nicht mehr als 30 K/h, z. B. 1 bis 30 K/h, häufig 2 bis 20 K/h und insbesondere 3 bis 15 K/h oder nicht kontrolliert erfolgen.

Es kann von Vorteil sein, im Anschluss and die Zugabe des Wassers oder nach Beendigung des Abkühlens den erhaltenen Niederschlag noch einige Zeit in der Mutterlauge zu suspendieren, z. B. über einen Zeitraum von 30 min. bis 3 d, bevor man das Kristallisat von der Mutterlauge abtrennt.

Alternativ kann man auch das Phenyluracil I, vorzugsweise die amorphe Form, in Wasser oder einem Gemisch aus Wasser und einem organischen Lösungsmittel suspendieren, wobei sich nach einiger Zeit ein efindungsgemäßes Hydrat des Phenyluracils I bildet. Der zuletzt genannte Weg ist insbesondere zur Herstellung der Form IV geeignet. Bezüglich der organischen Lösungsmittel gilt das zuvor Gesagte analog.

Die Gewinnung der Hydrate aus dem Kristallisationsgemisch, d. h. die Abtrennung der Hydrate von der Mutterlauge, gelingt durch übliche Techniken zur Trennung fester Bestandteile von Flüssigkeiten, z. B. durch Filtration, Zentrifugieren oder durch Dekantieren. In der Regel wird man den isolierten Feststoff waschen, z. B. mit dem zur Kristallisation verwendeten Lösungsmittel, mit Wasser oder mit einem Gemisch aus dem zur Kristallisation verwendeten organischen Lösungsmittel mit Wasser. Das Waschen kann in ein oder mehreren Schritten erfolgen, wobei man häufig im letzten Waschschritt mit Wasser wäscht. Das Waschen erfolgt typischerweise bei Temperaturen unterhalb 30 °C, häufig unterhalb 25 °C und insbesondere unterhalb 20 °C, um den Verlust an Wertprodukt möglichst gering zu halten. Anschließend kann man das erhaltene Hydrat schonend trocknen, um einen Verlust an Hydratwasser zu vermeiden, und dann der Weiterverarbeitung zuführen. Häufig wird man jedoch den nach Waschen erhaltenen feuchten Wirkstoff, insbesondere einen wasserfeuchten Wirkstoff direkt der weiteren Verarbeitung zuführen.

Durch die erfindungsgemäße Kristallisation wird ein Phenyluracil I erhalten, das im Wesentlichen aus einem efindungsgemäßen Hydrat besteht, d. h. der Anteil an Hydrat, bezogen auf die Gesamtmenge an Phenyluracil I im Kristallisationsgemisch liegt typischerweise bei wenigstens 90 %, häufig wenigstens 95 % und insbesondere wenigstens 98 %. Die Reinheit des Phenyluracils in den erhaltenen Hydraten, d. h. der Anteil an Phenyluracil I, bezogen auf die Gesamtmenge der im Hydrat enthaltenen organischen Bestandteile, beträgt in der Regel wenigstens 94 Gew.-%, insbesondere wenigstens 96 Gew.-%.

Die zur Kristallisation des Phenyluracils I eingesetzte Lösung kann z. B. durch folgende Methoden bereitgestellt werden:
(1) Lösen des Phenyluracils 1, vorzugsweise in einer von den Hydraten verschiedenen Form, in einem organischen Lösungsmittel oder Lösungsmittel-WasserGemisch; oder
(2) Herstellung des Phenyluracils I durch eine chemische Umsetzung und Überführung des Reaktionsgemischs, gegebenenfalls nach Abtrennen von Reagenzien und/oder Nebenprodukten, in ein erfindungsgemäß geeignetes organisches Lösungsmittel.

Zur Herstellung der Lösung durch Lösen des Phenyluracils I kann grundsätzlich jede bekannte Form des Phenyluracils I eingesetzt werden. Naturgemäß wird man eine Form des Phenyluracils I wählen, die von den Hydratformen verschieden ist. Hierzu kommen insbesondere eine feste oder flüssige Schmelze des Phenyluracils oder amorphes Phenyluracil I, wie aus dem Stand der Technik bekannt, in Betracht. Neben der amorphen Form I des Phenyluracils ist auch die kristalline Anhydrat-Form II des Phenyluracils geeignet. Die Anhydrat-Form II ist Gegenstand einer parallelen Patentanmeldung, auf die hier in vollem Umfang Bezug genommen wird.

Bei dem zum Lösen des Phenyluracils I verwendeten Lösungsmittel handelt es sich typischerweise um eines der vorgenannten organischen Lösungsmittel L1 oder um eine Mischung verschiedener Lösungsmittel L1 oder um ein Lösungsmittelgemisch das wenigstens 70 Gew.-% und speziell wenigstens 90 Gew.-%, bezogen auf die Gesamtmenge an dem zum Lösen eingesetzten Lösungsmittel, an Lösungsmittel L1 enthält.

Zum Lösen der von den Hydratformen III und IV verschiedenen Form des Phenyluracils I wird man üblicherweise das Phenyluracils I als feinteiligen Feststoff oder als Schmelze unter Durchmischen in das Lösungsmittel bei einer Temperatur einarbeiten, bei der das Lösungsmittel bzw. Lösungsmittelgemisch das Phenyluracil I vollständig zu Lösen vermag.

Das Lösen des Phenyluracils I folgt üblicherweise bei Temperaturen im Bereich von 20 bis 160 °C In einer bevorzugten Ausführungsform der Erfindung erfolgt das Lösen des Phenyluracil I bei erhöhter Temperatur, insbesondere bei wenigstens 50 °C, speziell bei wenigstens 80 °C, wobei die zum Lösen angewendete Temperatur naturgemäß den Siedepunkt des Lösungsmittels nicht überschreiten wird. Häufig erfolgt das Lösen bei Temperaturen im Bereich von 50 bis 140 °C, insbesondere im Bereich von 80 bis 120 °C und besonders bevorzugt im Bereich von 95 bis 115 °C.

Die Menge an Phenyluracil I, die im Lösungsmittel gelöst wird, hängt naturgemäß von der Art des Lösungsmittels L1 und der Lösungstemperatur ab und liegt häufig im Bereich von 50 bis 800 g/L. Geeignete Bedingungen können vom Fachmann durch Routineexperimente ermittelt werden.

Die Lösung des Phenyluracils I kann auch dadurch bereitgestellt werden, dass man ein durch eine chemische Umsetzung erhaltenes Reaktionsgemisch, welches das Phenyluracil I enthält, gegebenenfalls nach Abtrennen von Reagenzien und/oder Nebenprodukten, in ein erfindungsgemäß geeignetes organisches Lösungsmittel oder Lösungsmittel-Wasser-Gemisch überführt. Dabei kann man so vorgehen, dass man die Umsetzung in einem organischen Lösungsmittel oder Lösungsmittelgemisch durchführt, das zumindest teilweise, vorzugsweise zu wenigstens 50 Gew.-%, aus einem zur Kristallisation geeigneten Lösungsmittel besteht und, sofern erforderlich, eine Aufarbeitung durchführt, wobei man überschüssige Reagenzien und ggf. vorhandene Katalysatoren und ggf. vorhandenes, nicht geeignetes Lösungsmittel entfernt. Die Herstellung einer Lösung des Phenyluracils I durch chemische Umsetzung einer geeigneten Vorstufe des Phenyluracils I, kann in Analogie zu den Methoden, die im eingangs zitierten Stand der Technik beschrieben sind, erfolgen, worauf hier in vollem Umfang Bezug genommen wird.

Die Herstellung der Hydrate III und IV kann auch ausgehend von amorphem 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid erfolgen, indem man das amorphe Phenyluracil in Wasser oder einem wasserhaltigen organischen Lösungsmittel suspendiert. Als organische Lösungsmittel sind hierfür insbesondere solche Lösungsmittel L1 geeignet, die bei 298 K und 1 bar wenigstens 100 g/L Wasser zu lösen vermögen. Hierzu zählen insbesondere Dialkylketone mit 3 bis 5 C-Atomen, C₁-C₄-Alkanole, C₂C₃-Alkylenglykolmono-C₁-C₄-alkylether, Di-(C₂C₃-alkylenglykol-mono-C₁-C₄-alkyl-ether, Tetrahydrofuran und Gemischen dieser Lösungsmittel. Sofern eine Mischung aus Wasser und organischem Lösungsmittel eingesetzt wird, beträgt die Menge an Wasser typischerweise 50 bis 2000 Volumenteile, bezogen auf 100 Volumenteile organisches Lösungsmittel.

Die Herstellung des als Ausgangsmaterial für die Herstellung der Hydrate verwendeten 2-Chlor-5-[3,6-dihydro-2,6-dioxo-4-(trifluormethyl)-1 (2H)-pyrimidinyl]-4-tluor-N-[[methyl(1-methylethyl)-amino]sulfonyl]-benzamid kann nach den in WO 01/083459, WO 03/097589, WO 05/054208, WO 06/097589 und PCT/EP 2006/062414 beschriebenen Verfahren erfolgen, auf die hiermit in vollem Umfang Bezug genommen wird.

Besonders bevorzugt wird das Phenyluracil I nach folgenden Verfahren hergestellt:
1) Umwandlung der 2-Chlor-5-[3,6-dihydro-2,6-dioxo-4-(trifluormethyl)-1 (2H)-pyrimidinyl]-4-fluor-benzoesäure in ihr Säurechlorid oder das entsprechende Anhydrid und anschließende Umsetzung des entsprechenden aktivierten Säurederivats mit N-Methyl-N-(1-methylethyl)sulfamoylamid, z. B.: 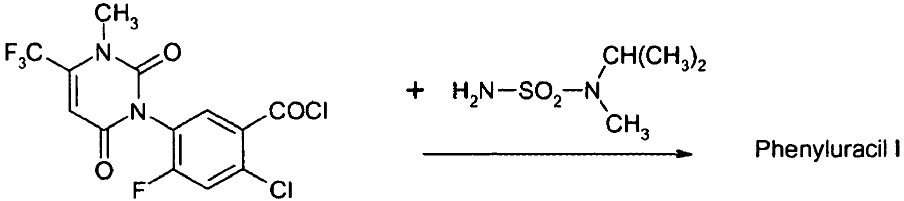
   Diese Umsetzung erfolgt üblicherweise bei Temperaturen von 20 °C bis zum Siedepunkt der Reaktionsmischung in einem organischen Lösungsmittel in Gegenwart einer Base sowie gegebenenfalls eines Katalysators [vgl. z. B. WO 01/083459, WO 03/097589 und auch WO 04/039768].
   Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropytether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid. Es können auch Gemische der genannten Lösungsmittel verwendet werden.
   Als Basen kommen allgemein anorganische Basen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z. B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht.
   Die Basen werden im Allgemeinen in katalytischen oder äquimolaren Mengen eingesetzt, sie können aber auch im Überschuss oder gegebenenfalls als Lösungsmittel verwendet werden.
   Die Edukte werden im Allgemeinen in äquimolaren Mengen miteinander umgesetzt. Es kann vorteilhaft sein, eines der Edukte in einem Überschuss einzusetzen.
2) Methylierung von 2-Chlor-5-[3,6-dihydro-2,6-dioxo-4-(trifluormethyl)-1 (2H)-pyrimidinyl]-4-fluor-N-[[methyl(1-methylethyl)amino]sulfonyl]-benzamid (im folgenden "NH-Uracil") mit einem Methylierungsmittel C:

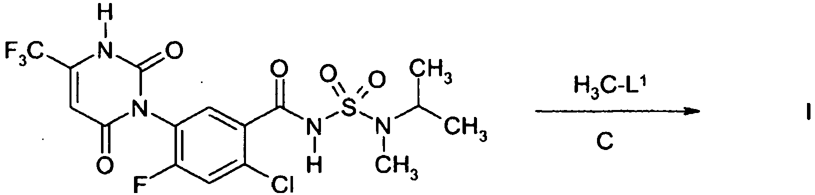

Die Gruppe L¹ steht für eine nucleophile Abgangsgruppe, bevorzugt für Halogen wie Chlor, Brom oder Iod, C₁-C₆-Alkylsulfat wie Methylsulfat, C₁-C₆-Alkylsulfonyloxy wie Methylsulfonyloxy, C₁-C₆-Halogenalkylsulfonyloxy wie Trifluormethylsulfonyloxy oder Phenylsulfonyloxy; sehr bevorzugt für C₁-C₆-Alkylsulfat.

Geeignete Methylierungsmittel C sind Methylhalogenide wie Methyliodid, Methylbromid, Methylchlorid , Dimethylsulfat, C₁-C₆-Halogenalkylsulfonsäure-methylester oder Phenylsulfonsäuremethylester, besonders bevorzugt sind Methylhalogenide und Dimethylsulfat; außerordentlich bevorzugt ist Dimethylsulfat.

Das Methylierungsmittel C kann sowohl in äquimolarer Menge, bezogen auf das NH-Uracil, als auch in substöchiometrischer Menge oder überstöchiometrischer Menge eingesetzt werden.

üblicherweise wird das Verfahren (2) in Gegenwart einer Base durchgeführt, wobei alle üblichen organischen und anorganischen Basen, z. B. die Verfahren (1) genannten Basen, in Betracht kommen. Bevorzugt sind Basen ausgewählt unter Alkali- und Erdalkalimetallhydroxiden wie Lithiumhydroxid, Natriumhydroxid, Kaliumhydroxid und Calciumhydroxid, Alkali- und Erdalkalimetalloxiden wie Lithiumoxid, Natriumoxid, Calciumoxid und Magnesiumoxid, Alkali- und Erdalkalimetallcarbonaten wie Lithiumcarbonat, Natriumcarbonat, Kaliumcarbonat und Calciumcarbonat sowie Alkalimetallhydrogencarbonaten wie Natriumhydrogencarbonat. In einer besonders bevorzugten Ausführungsform setzt man als Base Natrium- oder Kaliumhydroxid ein. Die Basen werden im Allgemeinen in äquimolaren Mengen bezogen auf das NH-Uracil eingesetzt, sie können aber auch katalytisch, im Überschuss oder gegebenenfalls als Lösungsmittel verwendet werden.

In einer sehr bevorzugten Variante des Verfahrens (2) wird der pH-Wert während der gesamten Umsetzung durch kontinuierliche oder portionsweise Basenzugabe in einem Bereich von 1 bis 6 gehalten. "Portionsweise Basenzugabe" bedeutet, dass die Basenzugabe während der Umsetzung in einzelnen Portionen erfolgt, d. h. in mindestens 2 Portionen, oder in mehreren bis zu vielen Portionen, oder kontinuierlich.

Zur Umsetzung können das NH-Uracil, das Methylierungsmittel C und gegebenenfalls die Base getrennt, gleichzeitig oder nacheinander in das Reaktionsgefäß eingebracht und zur Reaktion geführt werden.

Gemäß einer ersten Ausführungsform des Verfahrens (2) erfolgt die Umsetzung des NH-Uracils mit dem Methylierungsmittel C in einem organischen Lösungsmittel.

Als Lösungsmittel für diese Umsetzungen kommen, je nach Temperaturbereich, aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offfenkettige Dialkylether wie Diethylether, Di-n-proplyether, Di-n-isopropylether, Methyl-tert-butylether, cyclische Ether wie Tetrahydrofuran, 1,4-Dioxan, Anisol, Glykolether wie Dimethylglykolether, Diethylglykolether, Diethylenglykoldimethylether, Diethylenglykoldiethylether, C₁-C₄-Alkohole wie Methanol, Ethanol, n-Propanol, iso-Propanol, n-Butanol, aliphatische Carbonsäure-C₁-C₆-alkylester wie Methylacetat, Ethylacetat oder n-Butylacetat; Ketone wie Aceton, Methylethylketon, Methylisopropylketon, Methylisobutylketon, Butanon, Carbonate wie Diethylcarbonat und Ethylencarbonat, N,N-Dialkylamide wie N,N-Dimethylformamid oder N,N-Dimethylacetamid, N-Alkyllactame wie N-Methylpyrrolidon, Sulfoxide wie Dimethylsulfoxid, Tetraalkylharnstoffe wie Tetramethylharnstoff, Tetraethylharnstoff, Tetrabutylharnstoffe, Dimethylethylenharnstoff, Dimethylpropylenharnstoff oder Gemische dieser Lösungsmittel in Betracht.

Als Lösungsmittel bevorzugt sind N,N-Dialkylamide wie N,N-Dimethylformamid, N-Alkyllactame wie N-Methylpyrrolidon, Ketone wie Aceton, aromatische Kohlenwasserstoffe wie Toluol, chlorierte aliphatische und aromatische Kohlenwasserstoffe wie Dichlormethan oder Chlorbenzol, cyclische Ether wie Tetrahydrofuran, aliphatische Carbonsäure-C₁-C₆-alkylester wie Ethylacetat, Butylacetat oder Gemische dieser Lösungsmittel.

Vorzugsweise führt man die Methylierung des NH-Uracils bei Temperaturen zwischen -5 °C und 100 °C durch. Die Reaktionszeit kann der Fachmann in an sich üblicher Weise durch Routinemethoden wie Dünnschichtchromatographie oder HPLC ermitteln.

In einer anderen Variante des Verfahrens (2a) kann die Umsetzung auch in einem Mehrphasensystem durchgeführt werden. Diese Variante ist bevorzugt.

Bezüglich Methylierungsmittel C, pH-Wert, Base, Temperatur und Druck gilt das zuvor Gesagte.

Gemäß einer zweiten, bevorzugten Ausführungsform des Verfahrens (2) erfolgt die Umsetzung des NH-Uracils mit dem Methylierungsmittel C in einem wässrig-organischen Mehrphasensystem in Gegenwart eines oder mehrerer Phasentransferkatalysatoren.

Beispiele für Phasentransferkatalysatoren sind quartäre Ammoniumsalze, Phosphoniumsalze, Kronenether oder Polyglycole. Bevorzugte geeignete quartäre Ammoniumsalze umfassen z. B. Tetra-(C₁-C₁₈)-alkylammonium-halogenide und N-Benzyltri-(C₁-C₁₈)-alkylammonium-halogenide. Bevorzugte geeignete Phosphoniumsalze umfassen z. B. C₁-C₁₈-Alkyltriphenylphosphoniumchloride, -bromide, -acetate, Tetra-(C₁-C₁₈)-alkylphosphoniumchloride oder -bromide Tetraphenylphosphoniumchlorid oder -bromid, Benzyltriphenylphosphoniumchlorid oder-bromid. Bevorzugte geeignete Kronenether umfassen z. B. 18-Krone-6, Dibenzo-18-Krone-6. Bevorzugte geeignete Polyglycole umfassen z. B. Diethylenglycol-dibutylether (=Butyldiglyme), Tetraethylenglycol-dimethylether (= Tetraglyme), Triethylenglycol-dimethylether (= Triglyme), Polyglycol-dimethylether. In der Regel setzt man den Phasentransferkatalysator in einer Menge von bis zu 20 mol-%, bezogen auf das NH-Uracil ein.

Das Mehrphasensystem umfasst eine wässrige Phase und wenigstens eine organische flüssige Phase. Daneben können auch feste Phasen auftreten.

Die wässrige Phase ist vorzugsweise eine Lösung, welche die Base enthält, insbesondere eine wässrige Lösung von Alkali- oder Erdalkalihydroxiden (wie Lithiumhydroxid, Natriumhydroxid, Kaliumhydroxid und Calciumhydroxid), -carbonaten (wie Lithiumcarbonat, Natriumcarbonat, Kaliumcarbonat und Calciumcarbonat) oder Alkalimetallhydrogencarbonaten (wie Natriumhydrogencarbonat) in Wasser. Besonders bevorzugt verwendet man Alkali- oder Erdalkalihydroxide, sehr bevorzugt Natriumhydroxid.

Die Base(n) wird (werden) im Allgemeinen in äquimolaren Mengen bezogen auf das NH-Uracil eingesetzt, sie können aber auch katalytisch, im Überschuss oder gegebenenfalls als Lösungsmittel verwendet werden. Vorzugsweise setzt man wenigstens eine äquimolare Menge an Base, bezogen auf das NH-Uracil ein.

Für die organische Phase kommen vorzugsweise als Lösungsmittel je nach Temperaturbereich aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offenkettige Dialkylether wie Diethylether, Di-n-proplyether, Di-n-isopropylether, Methyl-tert-butylether, cyclische Ether wie Tetrahydrofuran (THF) und Anisol, aliphatische Carbonsäure-C₁-C₆-alkylester wie Methylacetat, Ethylacetat oder n-Butylacetat oder Gemische dieser Lösungsmittel in Betracht. Für die organische Phase sind Ethylacetat, n-Butylacetat, Chlorbenzol, THF, Toluol oder Gemische dieser Lösungsmittel als Lösungsmittel bevorzugt; sehr bevorzugt sind Ethylacetat, n-Butylacetat, Chlorbenzol und THF-Gemische sowie Toluol und THF-Gemische.

Während der Umsetzung können feste Phasen auftreten, wenn z. B. das NH-Uracil, das Methylierungsmittel C, die Base und/oder der Phasentransferkatalysator nicht vollständig gelöst sind.

In einer bevorzugten Ausführungsform besteht das Mehrphasensystem als wässrige Phase aus wässriger Natriumhydroxid-Lösung, und als organische Phase aus Toluol und Tetrahydrofuran, oder Dichlormethan und Tetrahydrofuran, Chlorbenzol und Tetrahydrofuran, oder aus Ethylacetat oder n-Butylacetat.

Zur Umsetzung können das NH-Uracil, die Methylierungsmittel C, die Base und der gegebenenfalls Phasentransferkatalysator getrennt, gleichzeitig oder nacheinander in das Reaktionsgefäß eingebracht und zur Reaktion geführt werden.

In der Regel wird man bei Verwendung eines Zweiphasensystems vor der Kristallisation der Form III bzw. IV die Phasen trennen. Besonders bevorzugt trocknet man das auf diesem Weg erhaltene Produkt vor der Kristallisation durch dem Fachmann bekannte Trocknungsmethoden, beispielsweise durch azeotropes Abdestillieren des Wasser zusammen mit einem Teil des organischen Lösungsmittels.

Die folgenden Abbildungen und Beispiele dienen der Erläuterung der Erfindung und sind nicht einschränkend zu verstehen.

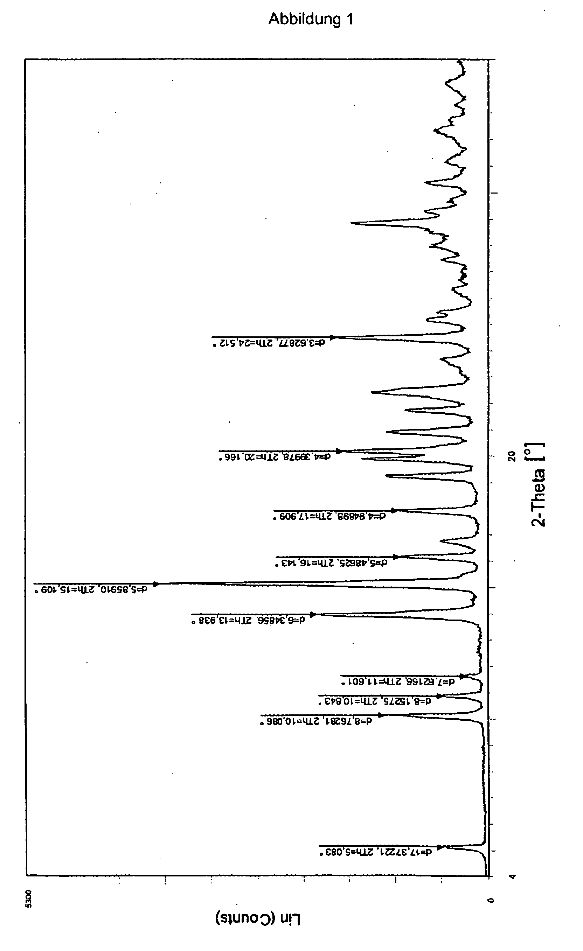

Abbildung 1 zeigt ein Röntgenpulverdifraktogramm der Form III. Das Röntgendiffraktogramm der Form III wurde mit einem Diffraktometer D-5000 der Fa. Bruker-AXS in Reflexionsgeometrie im Bereich von 2θ = 4° - 35° mit einer Schrittweite von 0,02° unter Verwendung der Cu-Kα-Strahlung bei 25°C aufgenommen. Aus ermittelten 2θ-Werten wurden die angegebenen Netzebenenabstände d berechnet.

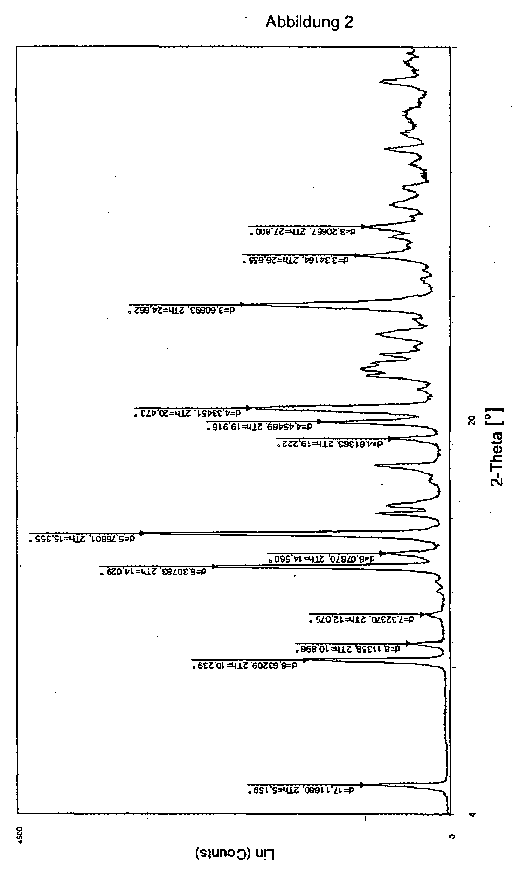

Abbildung 2 zeigt ein Röntgenpulverdifraktogramm der Form IV. Bezüglich der Messbedingungen gilt das zuvor Gesagte.

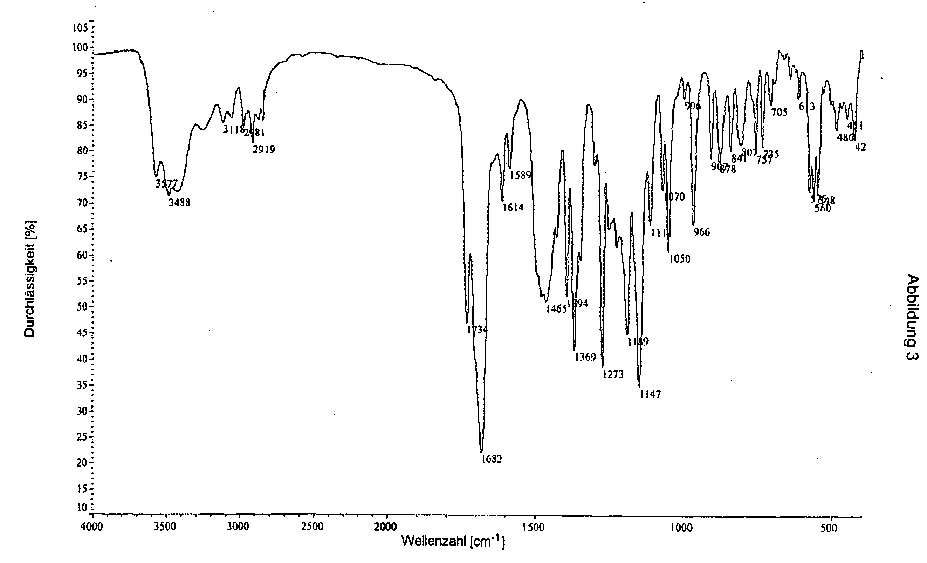

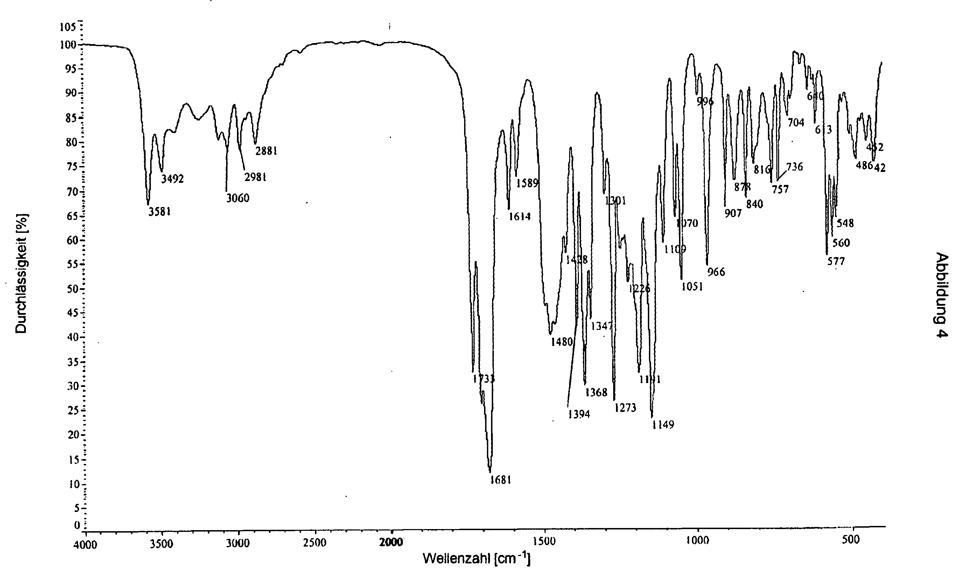

Abbildung 3 zeigt das IR-Spektrum der Form III und Abbildung 4 das der Form IV. Die IR-Spektren wurden mittels FTIR-Spectrometem "Nicolet Magna 550" und "Nicolet Magna 750" der Fa. Thermo Electron Corp./USA im Wellenzahlenbereich von 400 - 4000 cm⁻¹ bei einer Auflösung von 4 cm⁻¹ (32 Scans) aufgenommen. Als Probenkörper dienten KBr-Presslinge.

Die Bestimmung der Schmelzpunkte und Schmelzwärmen erfolgte mittels DSC mit einem Mettler Toledo DSC 25 der Fa. Mettler mit einer Aufheizrate von 5 K/min im Bereich von -5° bis +180 °C. Die Probenmenge betrug 5 bis 10 mg.

Die Bestimmung der kristallographischen Daten der Form III (Tabelle 2) erfolgte an einem Einkristalldiffraktometer der Fa. Bruker ("Bruker P4") unter Verwendung von Cu-Kα-Strahtung. Die Messung wurde bei -170 °C durchgeführt.

### Beispiel 1: Herstellung der Form III des Phenyluracils I durch Kristallisation der amorphen Form I aus Tetrahydrofuran/Wasser

Methode a: 20 g amorphes Phenyluracil wurde in 300 ml THF gelöst. Zu der Lösung gab man bei Raumtemperatur in einer Portion 300 ml Wasser und nach 48 h erneut weitere 300 ml Wasser. Die erhaltene Suspension wurde noch 3 Tage bei Raumtemperatur bewegt. Anschließend filtrierte man den erhaltenen Feststoff von der Mutterlauge. Das so erhaltene kristalline Material wurde mittels DSC und mittels Röntgenpulverdiffraktometrie (XRD) analysiert. Es wurde die Form III erhalten.

Methode b: 20 g amorphes Phenyluracil wurde in 300 ml THF gelöst. Man erwärmte auf 40 °C und gab zu der Lösung in einer Portion 300 ml Wasser. Anschließend kühlte man auf Raumtemperatur und filtrierte den erhaltenen Feststoff von der Mutterlauge. Das so erhaltene kristalline Material wurde mittels DSC und mittels Röntgenpulverdiffraktometrie (XRD) analysiert. Es wurde die Form III erhalten.

### Beispiel 2: Herstellung der Form III des Phenyluracils I durch Kristallisation der amorphen Form I aus Aceton/Wasser

2 g amorphes Phenyluracil wurde in 20 ml Aceton gelöst. Man erwärmte die Lösung auf 40 °C und gab hierzu unter Beibehaltung der Temperatur über einen Zeitraum von 2 h 5 Portionen Wasser à 5 ml. Hierbei begann ein Niederschlag auszukristallisieren. Man kühlte langsam auf Raumtemperatur (ca. 4 h) und filtrierte dann den erhaltenen Niederschlag ab. Ein Röntgenpulverdiffraktogramm bewies das Vorliegen der Form III.

### Beispiel 3: Herstellung der Form III des Phenyluracils I durch Kristallisation der amorphen Form I aus Ethylenglykolmonopropylether/Wasser

2 g amorphes Phenyluracil I wurden bei 80 °C in 20 ml Ethylenglykolmonopropylether gelöst. Man gab hierzu unter Beibehaltung der Temperatur über einen Zeitraum von 1 h 25 ml Wasser. Hierbei begann ein Niederschlag auszukristallisieren. Man kühlte langsam auf und filtrierte dann den erhaltenen Niederschlag ab. Ein Röntgenpulverdiffraktogramm bewies das Vorliegen der Form III.

### Beispiel 4: Herstellung der Form IV des Phenyluracils I durch Kristallisation der amorphen Form I aus Isopropanol/Wasser

5 g amorphes Phenyluracil wurden in 60 ml Isopropanol unter Erwärmen zum Rückfluss gelöst. Man gab hierzu unter Beibehaltung der Temperatur 50 ml Wasser. Hierbei begann ein Niederschlag auszukristallisieren. Man kühlte auf 50 bis 60 °C, behielt die Temperatur 2 Tage bei, kühlte dann auf Raumtemperatur und behielt die Temperatur 2 Tage bei. Anschließend erwärmte man auf 50 bis 60 °C, behielt die Temperatur 2 Tage bei, kühlte dann auf Raumtemperatur und filtrierte dann den erhaltenen Niederschlag ab. Das so erhaltene kristalline Material wurde mittels DSC und mittels Röntgenpulverdiffraktometrie (XRD) analysiert. Es wurde die Form IV erhalten.

### Beispiel 5: Herstellung der Form IV des Phenyluracils I durch Umfällen der amorphen Form I in Wasser

2 g amorphes Phenyluracil I wurden 2 Tage bei Raumtemperatur in 20 ml Wasser gerührt. Dann wurde der Feststoff mittels Zentrifugation abgetrennt. Ein Röntgenpulverdiffraktogramm bewies das Vorliegen der Form IV.

### Beispiel 6: Herstellung der Form IV des Phenyluracils I durch Kristallisation der amorphen Form I aus Tetrahydrofuran/Wasser

Methode a: 20 g amorphes Phenyluracil I wurden in 300 ml THF gelöst. Zu der Lösung gab man bei Raumtemperatur in einer Portion 600 ml Wasser. Die erhaltene Suspension wurde noch 2 Tage bei Raumtemperatur bewegt. Anschließend filtrierte man den erhaltenen Feststoff von der Mutterlauge. Ein Röntgenpulverdiffraktogramm bewies das Vorliegen der Form IV.

### Beispiel 7: Herstellung der Form 111 durch Umsetzung von N-(2-Chlor-4-fluor-5-isocyanatobenzoyl)-N'-methyl-(1-methylethyl)sulfamid mit 3-Methylamino-4,4,4-trifluorcrotonsäureethylester und Präzipitation aus Methanol/Wässer

Unter Stickstoff rührte man 0,99 g (5,021 mmol) 3-Methylamino-4,4,4-trifluorcrotonsäureethylester in 25 ml N,N-Dimethylformamid und 50 ml n-Pentan 45 Minuten am Wasserabscheider am Rückfluss. Anschließend destillierte man das n-Pentan bis zu einer Innentemperatur von 70 °C ab. Man ließ auf 40 °C abkühlen und gab danach innerhalb 15 Minuten bei einer Temperatur von bis zu 45 °C 1,13 g (10,043 mmol) Kalium-tert-butylat in 3 Portionen unter Rühren zu, wobei eine rotbraune Lösung entstand. Nach 20minütigem Rühren bei 40 °C ließ man abkühlen und gab dann bei -15 °C bis -10 °C 1,55 g (4,419 mmol) N-(2-Chlor-4-fluor-5-isocyanatobenzoyl)-N'-methyl-(1-methylethyl)sulfamid innerhalb 2 Minuten zu, wobei sofortige Lösung eintrat. Man rührte 30 Minuten bei -10 °C, ließ danach das Reaktionsgemisch auf 22 °C erwärmen und rührte noch 30 Minuten bei dieser Temperatur nach.

Unter leichtem Kühlen bei 20 - 22 °C säuerte man das erhaltene Reaktionsgemisch mit 0,46 g (12,553 mmol) 4 n Salzsäure in 3,1 ml Dioxan an, wobei ein Niederschlag ausfiel, und engte im Vakuum ein. Den erhaltenen Rückstand verteilte man in einem Lösungsmittelgemisch aus 100 ml Methyl-tert.-butylether und 100 ml Wasser. Die organische Phase trennte man ab und engte danach im Vakuum bis zur Trockne ein. Das glasartige Harz nahm man in einem eiskalten Gemisch aus Methanol:Wasser = 7 : 3 unter Rühren auf, wobei innerhalb 30 min Niederschlagsbildung einsetzte. Den abgesaugten Rückstand rührte man bei 0 °C 0,5 h in Methyl-tert.-butylether, saugte ab, wusch mit Methyl-tert.-butylether und trocknete im Vakuum, wobei man 1,00 g (43,6 % der Theorie) der Titelverbindung als farbloses Pulver mit einer ¹H-NMR-Reinheit von 95 % und einem Schmelzpunkt von 107 -122 °C erhielt. Ein Röntgenpulverdiffraktogramm bewies das Vorliegen der Form III.

### Beispiel 8: Herstellung der Form III durch Präzipitation des bei der Methylierung anfallenden Rohproduktes aus Methyl-tert.-butylether und Wasser

Zu einem Lösungsmittelgemisch aus 155 g Toluol und 31 g Tetrahydrofuran gab man bei 25 °C 14,18 g (0,0274 mol) 2-Chlor-5-[3,6-dihydro-2,6-dioxo-4-(trifluormethyl)-1(2H)-pyrimidinyl]-4-fluor-N-[[methyl(1-methylethyl)amino]sulfonyl]benzamid (93,9%ig) und versetzte danach das Gemisch mit einer Lösung aus 2,55 g (0,0319 mol) Natriumhydroxid (50%ig) in 61,2 g Wasser. Zu dem Reaktionsgemisch gab man 0,88 g (0,0027 mol) Tetrabutylammoniumbromid und 4,08 g (0,0329 mol) Dimethylsulfat zu. Das zweiphasige Reaktionsgemisch rührte man 23 Stunden intensiv bei 25 °C. Danach trennte man die wässrige Phase ab und wusch die organische Phase zweimal mit jeweils 100 ml Wasser. Nach dem Trocknen der vereinten organischen Phase destillierte man das Lösungsmittel bei reduziertem Druck ab, wobei man 15.4 g eines rohen Produktes erhielt, welches nach quantitativer HPLC die Titelverbindung zu 77,6 % enthielt (entspricht einer Ausbeute von 87,2 %).

Danach gab man aber zu einem auf 40 °C erwärmten Lösungsmittelgemisch aus 60 ml Methyl-tert-butylether und 6 ml Wasser unter Rühren 14 g des erhaltenen rohen Produktes. Man ließ langsam auf 0 °C abkühlen, wobei ein Niederschlag ausfiel. Den ausgefallenen Feststoff saugte man ab und trocknete ihn. Bei diesem Fällungsschritt erhielt man 11,3 g der Titelverbindung (Reinheit nach quantitativer HPLC bestimmt als Monohydrat: 93,3%; Gesamtausbeute nach Fällung: 81,5 %). Ein Röntgenpulverdiffraktogramm bewies das Vorliegen der Form III.

### Beispiel 9: Herstellung des kristallinen Hydrates Form III durch Kristallisation aus Toluol/Wasser

Eine Lösung von 0,92 mol des 2-Chlor-5-[3,6-dihydro-3-methyl-2,6-dioxo-4-trifluormethyl-1-(2H)-pyrimidinyl]-4-fluor-N-[(methyl-isopropyl-amino)sulfonyl]-benzamids in 95 % Toluol mit 5 % THF wurde mit 180 g (10 mol) Wasser bei 75 °C versetzt und innerhalb von 3 h auf 20 °C abgekühlt. Es wurde 15 h nachgerührt und der ausgefallene Feststoff wurde bei 20 °C abfiltriert. Der Feststoff wurde auf dem Filter mit 150 g Toluol gewaschen und im Vakuum bei Temperaturen < 50 °C getrocknet. Ausbeute: 0,82 mol. Ein Röntgenpulverdiffraktogramm bewies das Vorliegen der Form III.

### Beispiel 10: Herstellung des kristallinen Hydrats von 2-Chlor-5-[3,6-dihydro-3-methyl-2,6-dioxo-4-trifluormethyl-1-(2H)-pyrimidinyl]-4-fluor-N-[(methyl-isopropyl-amino)-sulfonyl]-benzamid (= Form III) aus der Reaktionslösung

50,0 g (0,098 mol) 2-Chlor-5-[3,6-dihydro-2,6-dioxo-4-(trifluormethyl)-1 (2H)-pyrimidinyl]-4-fluor-N-{[methyl-(1-methylethyl)amino]sulfonyl}-benzamid, 3,2 g (0,0089 mol) Tetrabutylammoniumbromid (= TBAB) und 15,1 g (0,12 mol) Dimethylsulfat wurden bei 25 °C in einem Gemisch aus Toluol, Wasser und THF vorgelegt und der Ansatz auf 40 °C aufgeheizt. Anschließend stellte man durch Zugabe von wässriger 10%iger NaOH-Lösung im Reaktionsgemisch einen pH-Wert von 5,3 - 5,5 ein. Während der ganzen Reaktionsdauer wurde weiterhin wässrige 10%ige NaOH-Lösung zugegeben, so dass der pH-Wert über den gesamten Reaktionsverlauf konstant bei dem zuvor eingestellten pH-Wert lag. Nach beendeter Reaktion wurde das Reaktionsgemisch noch 3,5 h bei 40 °C nachgerührt.
Methode a): Die Phasen wurden bei 40 °C getrennt. Man gab 250 g Wasser zur organischen Phase und destillierte das Toluol und das THF vollständig azeotrop ab. Die resultierende Mischung wurde bei 65 °C mit Methanol versetzt und innerhalb von 3 h auf 20 °C abgekühlt. Man erhielt 45.6 g (82 % der Theorie; Reinheit 91,4 %) des Phenyluracils als Form III, welche aufgrund ihres Röntgenpulverdiffraktogramms identifiziert wurde.
Methode b): Man trennte die Phasen und destillierte 65 bis 70 % des eingesetzten Lösungsmittels ab. Man kühlte die Lösung auf 75 °C ab und gab anschließend 18 g Wasser zu. Die Lösung wurde innerhalb von 3 h linear auf 20 °C abgekühlt und 3 h bei 20 °C nachgerührt. Der ausgefallene Feststoff wurde abgesaugt und getrocknet. Man erhielt 44,8 g (83 % der Theorie; Reinheit 94,1 %) der Form III, welche aufgrund ihres Röntgenpulverdiffraktogramms identifiziert wurde.

Die Formen III und IV eignen sich ebenso wie die Form I als Herbizid, sind jedoch dieser bezüglich Wirksamkeit überlegen. Die Erfindung betrifft daher auch Pflanzenschutzmittel, enthaltend die kristalline Form III bzw. Form IV und für die Formulierung von Pflanzenschutzmitteln übliche Hilfsmittel, insbesondere Pflanzenschutzmittel in Form von wässrigen oder nicht-wässrigen Suspensionskonzentraten. Die Erfindung betrifft auch ein Verfahren zur Bekämpfung von unerwünschtem Pflanzenwuchs, das dadurch gekennzeichnet ist, dass man die Form III bzw. Form IV des Phenyluracils, vorzugsweise als geeignete Wirkstoffaufbereitung auf Pflanzen, deren Lebensraum und/oder auf Samen einwirken lässt.

Die die Form III bzw. Form IV enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode kann die Form III bzw. Form IV bzw. können die sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus armeniaca, Prunus avium, Prunus cerasus, Prunus dulcis, Prunus domesticua, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus kann die Form III bzw. Form IV bzw. können die sie enthaltenden herbiziden Mittel auch in Kulturen, die durch Züchtung einschließlich gentechnischer Methoden gegen die Wirkung von Herbiziden tolerant sind, verwendet werden.

Weiterhin kann die Form III bzw. Form IV bzw. können die sie enthaltenden herbiziden Mittel auch in Kulturen, die durch Züchtung einschließlich gentechnischer Methoden gegen Insekten- oder Pilzbefall tolerant sind, verwendet werden.

Des Weiteren wurde gefunden, dass die Formen III und IV auch zur Defoliation und Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Form III bzw. der Form IV gefunden.

Als Desikkantien eignet sich die Formen III und IV insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht. Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein - und Schalenobst ermöglicht wird. Derselbe Mechanismus, d. h., die Förderung der Ausbildung von Trenngewebe zwischen Frucht- oder Blatt- und Sprossteil der Pflanzen ist auch für ein gut kontrollierbares Entblättem von Nutzpflanzen, insbesondere Baumwolle, wesentlich. Außerdem führt die Verkürzung des Zeitintervalls, in dem die einzelnen Baumwollpflanzen reif werden, zu einer erhöhten Faserqualität nach der Ernte.

Des Weiteren wurde gefunden, dass die Formen III und IV auch zur Kontrolle von Koniferen, insbesondere von wildwachsenden Koniferensämlingen, speziell zur Kontrolle von wildwachsenden Kiefernsämlingen geeignet sind.

Weiterhin eignen sich die Formen III und IV auch zur Kontrolle von Unkräutern in Kulturpflanzen, wie z. B. Sojabohne, Baumwolle, Raps, Flachs, Linsen, Reis, Zuckerrübe, Sonnenblume, Tabak und Getreide, wie z. B. Mais oder Weizen.

Die Form III bzw. IV bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen, Ölsuspensionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge der Form III bzw. IV und für die Formulierung von Pflanzenschutzmitteln übliche Hilfsmittel und Trägerstoffe.

Als Trägerstoffe kommen grundsätzlich alle festen Substanzen in Betracht, die üblicherweise in Pflanzenschutzmitteln, insbesondere in Herbiziden zum Einsatz kommen. Feste Trägerstoffe sind z. B. Mineralerden, wie Kieselgele, Silikate, Talkum, Kaolin, Attaclay, Kalkstein, Kalk, Kreide, Bolus, Löß, Ton, Dolomit, Diatomeenerde, Calcium - und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Im Falle von flüssigen Formulierungen der Form III bzw. IV weisen die Zusammensetzungen eine flüssige Phase auf. Als flüssige Phase kommen grundsätzlich Wasser sowie solche organischen Lösungsmittel in Betracht, in denen die Formen III bzw. IV nur eine geringe oder keine Löslichkeit besitzt, z. B. solche, in denen die Löslichkeit der Formen III bzw. IV des Phenyluracils I bei 25 °C und 1013 mbar nicht mehr als 1 Gew.-%, insbesondere nicht mehr als 0,1 Gew.-% und speziell nicht mehr als 0,01 Gew.-% beträgt.

Bevorzugte flüssige Phasen sind insbesondere Wasser und wässrige Lösungsmittel, d. h. Lösungsmittelgemische, die neben Wasser noch bis zu 30 Gew.-%, vorzugsweise jedoch nicht mehr als 10 Gew.-%, bezogen auf die Gesamtmenge an Wasser und Lösungsmittel, eines oder mehrere mit Wasser mischbare organische Lösungsmittel, z. B. mit Wasser mischbare Ether wie Tetrahydrofuran, Methylglykol, Methyldiglykol, Alkanole wie Methanol, Ethanol, Isopropanol oder Polyole wie Glykol, Glycerin, Diethylenglykol, Propylenglykol und dergleichen, enthalten.

Bevorzugte flüssige Phasen sind weiterhin nicht-wässrige organische Lösungsmittel, in denen die Löslichkeit der Form III bzw. IV des Phenyluracils I bei 25 °C und 1013 mbar nicht mehr als 1 Gew.-%, insbesondere nicht mehr als 0,1 Gew.-% und speziell nicht mehr als 0,01 Gew.-% beträgt. Hierzu zählen insbesondere aliphatische und cycloaliphatische Kohlenwasserstoffe sowie Öle, insbesondere solche pflanzlichen Ursprungs, weiterhin C₁-C₄-Alkylester von gesättigten oder ungesättigten Fettsäuren oder Fettsäuregemischen, insbesondere die Methylester, z. B. Ölsäuremethylester, Stearinsäuremethylester, Rapsölmethylester, aber auch paraffinische Mineralöle und dergleichen.

Typische Hilfsmittel umfassen oberflächenaktive Substanzen, insbesondere die in Pflanzenschutzmitteln üblicherweise eingesetzten Netzmittel und Dispergier(hilfs)mittel, weiterhin die Viskosität verändernde Additive (Verdicker, Andicker), Antischaummittel, Frostschutzmittel, Mittel zur Einstellung des pH-Wertes, Stabilisatoren, Anticaking-Mittel und Biozide (Konservierungsmittel).

Die Erfindung betrifft insbesondere Mittel für den Pflanzenschutz in Form eines wässrigen Suspensionskonzentrats (SC). Derartige Suspensionskonzentrate enthalten die Form III bzw. IV des Phenyluracils I in einer feinteiligen partikulären Form, wobei die Partikel der Form III bzw. IV in einer wässrigen Phase suspendiert vorliegen. Die Größe der Wirkstoffpartikel, d. h. die Größe, welche 90 Gew.-% der Wirkstoffpartikel nicht überschreiten, liegt dabei typischerweise unterhalb 30 µm, insbesondere unterhalb 20 µm. Vorteilhafterweise weisen in den erfindungsgemäßen SC's wenigstens 40 Gew.-% und insbesondere wenigstens 60 Gew.-% der Teilchen Durchmesser unterhalb 2 µm auf.

Wässrige Suspensionskonzentrate enthalten neben dem Wirkstoff typischerweise oberflächenaktive Substanzen, sowie gegebenenfalls Antischaummittel, Verdicker (= Andicker), Frostschutzmittel, Stabilisatoren (Biozide), Mittel zur Einstellung des pH-Wertes und Anticaking-Mittel.

Die Menge an Wirkstoff, d. h. die Gesamtmenge an Phenyluracil der Form III bzw. IV sowie an gegebenenfalls weiteren Wirkstoffen, liegt in derartigen SC's üblicherweise im Bereich von 10 bis 70 Gew.-%, insbesondere im Bereich von 20 bis 50 Gew.-%, bezogen auf das Gesamtgewicht des Suspensionskonzentrats.

Als oberflächenaktive Substanzen kommen vorzugsweise anionische und nichtionische Tenside in Betracht. Geeignete oberflächenaktive Substanzen sind auch Schutzkolloide. Die Menge an oberflächenaktiven Substanzen wird in der Regel 0,5 bis 30 Gew.-%, insbesondere 1 bis 20 Gew.-%, bezogen auf das Gesamtgewicht der erfindungsgemäßen wässrigen SC's betragen. Vorzugsweise umfassen die die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 10: 1 bis 1 : 10 liegt.

Zu den Beispielen anionischer oberflächenaktiver Substanzen (Tenside) zählen Alkylarylsulfonate, Phenylsulfonate, Alkylsulfate, Alkylsulfonate, Alkylethersulfate, Alkylarylethersulfate, Alkylpolyglykoletherphosphate, Polyarylphenyletherphosphate, Alkylsulfosuccinate, Olefinsulfonate, Paraffinsulfonate, Petroleumsulfonate, Tauride, Sarkoside, Fettsäuren, Alkylnaphthalinsulfonsäuren, Naphthalinsulfonsäuren, Ligninsulfonsäuren, Kondensationsprodukte sulfonierter Naphthaline mit Formaldehyd oder mit Formaldehyd und Phenol und gegebenenfalls Harnstoff sowie Kondensationsprodukte aus Phenolsulfonsäure, Formaldehyd und Harnstoff, Lignin-Sulfit-Ablauge und Ligninsulfonate, Alkylphosphate, Alkylarylphosphate, z. B. Tristyrylphosphate, sowie Polycarboxylate wie z. B. Polyacrylate, Maleinsäureanhydrid/Olefin-Copolymere (z. B. Sokalan^{®} CP9, BASF), einschließlich der Alkali-, Erdalkali-, Ammonium- und Amin-Salze der vorgenannten Substanzen. Bevorzugte anionische oberflächenaktive Substanzen sind solche, die wenigstens eine Sulfonatgruppe tragen und insbesondere deren Alkalimetall - und deren Ammoniumsalze.

Beispiele für nichtionische oberflächenaktiver Substanzen umfassen Alkylphenolalkoxylate, Alkoholalkoxylate, Fettaminalkoxylate, Polyoxyethylenglycerolfettsäureester, Rizinusölalkoxylate, Fettsäurealkoxylate, Fettsäureamidalkoxylate, Fettsäurepolydiethanolamide, Lanolineth-oxylate, Fettsäurepolyglykolester, Isotridecylalkohol, Fettsäureamide, Methylcellulose, Fettsäureester, Alkylpolyglykoside, Glycerolfettsäureester, Polyethylenglykol, Polypropylenglykol, Polyethylenglykolpolypropylenglykol-Blockcopolymere, Polyethylenglykolalkylether, Polypropylenglykolalkylether, Polyethylenglykolpolypropylenglykolether-Blockcopolymere (Polyethylenoxid-Polypropylenoxid-Blockcopolymere) und deren Gemische. Bevorzugte nichtionische oberflächenaktive Substanzen sind Fettalkoholethoxylate, Alkylpolyglykoside, Glycerolfettsäureester, Rizinusölalkoxylate, Fettsäurealkoxylate, Fettsäureamidalkoxylate, Lanolinethoxylate, Fettsäurepolyglykolester und Ethylenoxid-Propylenoxid-Blockcopolymere und deren Mischungen.

Schutzkolloide sind typischerweise wasserlösliche, amphiphile Polymere. Beispiele hierfür sind Proteine und denaturierte Proteine wie Casein, Polysaccharide wie wasserlösliche Stärkederivate und Cellulosederivate, insbesondere hydrophob modifizierte Stärken und Cellulosen, weiterhin Polycarboxylate wie Polyacrylsäure und Acrylsäurecopolymere, Polyvinylalkohol, Polyvinylpyrrolidon, Vinylpyrrolidon-Copolymere, Polyvinylamine, Polyethylenimine, und Polyalkylenether.

Für die erfindungsgemäßen wässrigen SC's geeignete, die Viskosität verändernde Additive (Andicker) sind insbesondere Verbindungen, die der Formulierung ein modifiziertes Fließverhalten verleihen, z.B. eine hohe Viskosität im Ruhezustand und niedrige Viskosität im bewegten Zustand. Geeignet sind grundsätzlich alle für diesen Zweck in Suspensionskonzentraten eingesetzte Verbindungen. Zu nennen sind beispielsweise anorganische Substanzen, z.B. Schichtsilikate und organisch modifizierte Schichtsilikate wie Bentonite oder Attapulgite (z. B. Attaclay^{®} Firma Engelhardt), und organische Substanzen wie Polysaccharide und Heteropolysaccharide wie Xanthan Gum^{®} (Kelzan^{®} der Fa. Kelco), Rhodopol^{®} 23 (Rhone Poulenc) oder Veegum^{®} (Firma R.T. Vanderbilt) zu nennen, wobei Xanthan-Gum^{®} bevorzugt verwendet wird. Die Menge der die Viskosität verändernden Additive beträgt häufig 0,1 bis 5 Gew.-%, bezogen auf das Gesamtgewicht des SC's.

Als für die erfindungsgemäßen wässrigen SC's geeignete Antischaummittel kommen beispielsweise für diesen Zweck bekannte Silikonemulsionen (Silikon^{®} SRE, Firma Wacker oder Rhodorsil^{®} der Firma Rhodia), langkettige Alkohole, Fettsäuren und deren Salze, Entschäumer vom Typ wässriger Wachsdispersionen, feste Entschäumer (sog. Compounds), fluororganische Verbindungen und deren Gemische in Betracht. Die Menge an Antischaummittel beträgt typischerweise 0,1 bis 1 Gew.-%, bezogen auf das Gesamtgewicht des SC's.

Den erfindungsgemäßen Suspensionskonzentraten kann man zur Stabilisierung auch Konservierungsmittel zusetzen. Geeignete Konservierungsmittel sind solche auf Basis von Isothiazolonen, beispielsweise Proxel^{®} der Fa. ICI oder Acticide^{®} RS der Fa. Thor Chemie oder Kathon^{®} MK der Firma Rohm & Haas. Die Menge an Konservierungsmittel beträgt typischerweise 0,05 bis 0,5 Gew.-%, bezogen auf das Gesamtgewicht des SC's.

Geeignete Frostschutzmittel sind flüssige Polyole, z. B. Ethylenglycol, Propylenglycol oder Glycerin sowie Harnstoff. Die Menge an Frostschutzmitteln beträgt in der Regel 1 bis 20 Gew.-%, insbesondere 5 bis 10 Gew.-%, bezogen auf das Gesamtgewicht des wässrigen Suspensionskonzentrats.

Gegebenenfalls können die erfindungsgemäßen wässrigen SC's Puffer zur pH-Wert Regulation enthalten. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

Sofern die Formulierungen der Form III bzw. IV zur Saatgutbehandlung eingesetzt werden, können sie weitere übliche Bestandteile enthalten, wie sie bei der Saatgutbehandlung, z. B. bei Beizen oder Coaten eingesetzt werden. Hierzu zählen neben den vorgenannten Bestandteilen insbesondere Farbmittel, Kleber, Füllstoffe und Weichmacher.

Als Farbmittel kommen alle die für derartige Zwecke üblichen Farbstoffe und Pigmente in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C. I. Pigment Red 112 und C. I. Solvent Red 1, Pigment blue 15:4, Pigment blue 15:3, Pigment blue 15:2, Pigment blue 15:1, Pigment blue 80, Pigment yellow 1, Pigment yellow 13, Pigment red 48:2, Pigment red 48:1, Pigment red 57:1, Pigment red 53:1. Pigment orange 43. Pigment orange 34. Pigment orange 5. Pigment green 36, Pigment green 7, Pigment white 6, Pigment brown 25, Basic violet 10, Basic violet 49, Acid red 51, Acid red 52, Acid red 14, Acid blue 9, Acid yellow 23, Basic red 10, Basic red 108 bekannten Farbstoffe und Pigmente. Die Menge an Farbmittel wird üblicherweise nicht mehr als 20 Gew.-% der Formulierung ausmachen und liegt vorzugsweise im Bereich von 0,1 bis 15 Gew.-%, bezogen auf das Gesamtgewicht der Formulierung.

Als Kleber kommen alle üblichen in Beizmitteln einsetzbaren Bindemittel in Frage. Beispiele für geeignete Bindemittel umfassen thermoplastische Polymere wie Polyvinylpyrrolidon, Polyvinylacetat, Polyvinylalkohol und Tylose weiterhin Polyacrylate, Polymethacrylate, Polybutene, Polyisobutene, Polystyrol, Polyethylenamine, Polyethylenamide, die vorgenannten Schutzkolloide, Polyester, Polyetherester, Polyanhydride, Polyesterurethane, Polyesteramide, thermoplastische Polysaccharide, z. B. Cellulosederivate wie Celluloseester, Celluloseether, Celluloseetherester, einschließlich Methylcellulose, Ethylcellulose, Hydroxymethylcellulose, Carboxymethylcellulose, Hydroxypropylcellulose und Stärkederivate und modifizierte Stärken, Dextrine, Maltodextrine, Alginate und Chitosane, weiterhin Fette, Öle, Proteine, einschließlich Casein, Gelatine und Zein, Gummi-Arabicum, Schellack. Vorzugsweise sind die Kleber pflanzenverträglich, d. h. sie weisen keine oder keinen nennenswerten phytotoxischen Wirkungen auf. Vorzugsweise sind die Kleber biologisch abbaubar. Vorzugsweise ist der Kleber so gewählt, dass er als Matrix für die aktiven Bestandteile der Formulierung wirkt. Die Menge an Kleber wird üblicherweise nicht mehr als 40 Gew.-% der Formulierung ausmachen und liegt vorzugsweise im Bereich von 1 bis 40 Gew.-% und insbesondere im Bereich von 5 bis 30 Gew.-%, bezogen auf das Gesamtgewicht der Formulierung.

Neben dem Kleber kann die Formulierung für die Saatgutbehandlung auch inerte Füllstoffe enthalten. Beispiele hierfür sind die vorgenannten festen Trägermaterialien, insbesondere feinteilige anorganische Materialien wie Tone, Kreide, Bentonit, Kaolin, Talkum, Perlit, Mica, Kieselgel, Diatomeenerde, Quarzpulver, Montmorillonit, aber auch feinteilige organische Materialen, wie Holzmehl, Getreidemehl, Aktivkohle und dergleichen. Die Menge an Füllstoff wird vorzugsweise so gewählt, dass die Gesamtmenge an Füllstoff 75 Gew.-%, bezogen auf das Gesamtgewicht aller nicht flüchtigen Bestandteile der Formulierung nicht überschreitet. Häufig liegt die Menge an Füllstoff im Bereich von 1 bis 50 Gew.-%, bezogen auf das Gesamtgewicht aller nicht flüchtigen Bestandteile der Formulierung.

### Daneben kann die Formulierung für die Saatgutbehandlung noch einen Weichmacher enthalten, der die Flexibilität der Beschichtung erhöht. Beispiele für Weichmacher sind oligomere Polyalkylenglykole, Glycerin, Dialkylphthalate, Alkylbenzylphthalate, Glykolbenzoate und vergleichbare Verbindungen. Die Menge an Weichmacher in der Beschichtung liegt häufig im Bereich von 0,1 bis 20 Gew.-%, bezogen auf das Gesamtgewicht aller nicht flüchtigen Bestandteile der Formulierung.

Die Erfindung betrifft insbesondere auch Mittel für den Pflanzenschutz in Form eines nicht-wässrigen Suspensionskonzentrats. Derartige Suspensionskonzentrate enthalten die Form III bzw. die Form IV des Phenyluracils I in einer feinteiligen partikulären Form, wobei die Partikel der Form III bzw. IV in einer nicht-wässrigen Phase suspendiert vorliegen. Die Größe der Wirkstoffpartikel, d. h. die Größe, welche 90 Gew.-% der Wirkstoffpartikel nicht überschreiten, liegt dabei typischerweise unterhalb 30 µm, insbesondere unterhalb 20 µm. Vorteilhafterweise weisen in den nicht-wässrigen SC's wenigstens 40 Gew.-% und insbesondere wenigstens 60 Gew.-% der Teilchen Durchmesser unterhalb 2 µm auf.

Nicht-wässrige Suspensionskonzentrate enthalten neben dem Wirkstoff typischerweise oberflächenaktive Substanzen, sowie gegebenenfalls Antischaummittel, Verdicker (= Andicker), Frostschutzmittel, Stabilisatoren (Biozide), Mittel zur Einstellung des pH-Wertes und Anticaking-Mittel.

Die Menge an Wirkstoff, d. h. die Gesamtmenge an Phenyluracil I in der Form III bzw. IV sowie an gegebenenfalls weiteren Wirkstoffen, liegt in den nicht-wässrigen SC's üblicherweise im Bereich von 10 bis 70 Gew.-%, insbesondere im Bereich von 20 bis 50 Gew.-%, bezogen auf das Gesamtgewicht des nicht-wässrigen Suspensionskonzentrats.

Als oberflächenaktive Substanzen kommen vorzugsweise die zuvor genannten anionischen und nichtionischen Tenside in Betracht. Die Menge an oberflächenaktiven Substanzen wird in der Regel 1 bis 30 Gew.-%, insbesondere 2 bis 20 Gew.-%, bezogen auf das Gesamtgewicht der erfindungsgemäßen nicht-wässrigen SC's betragen. Vorzugsweise umfassen die die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 10 : 1 bis 1 : 10 liegt.

Die erfindungsgemäßen Formen III und IV können auch als Pulver-, Streu- und Stäubemittel formuliert werden. Derartige Formulierungen können durch Mischen oder gemeinsames Vermahlen der Form III bzw. IV mit einem festen Trägerstoff und gegebenenfalls weiteren Hilfsmitteln hergestellt werden.

Die erfindungsgemäßen Form III und IV können auch in Form von Granulaten, z. B. Umhüllungs-, Imprägnierungs- und Homogengranulaten formuliert werden. Derartige Formulierungen können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe.

Die Konzentrationen der Form III bzw. IV in den anwendungsfertigen Zubereitungen können in weiten Bereichen variiert werden. Im Allgemeinen enthalten die Formulierungen etwa von 11 bis 98 Gew.-%, vorzugsweise 10 bis 95 Gew.-%, bezogen auf das Gesamtgewicht an Wirkstoffen.

Die folgenden Formulierungsbeispiele verdeutlichen die Herstellung solcher Zubereitungen:
I. 20 Gewichtsteile der Form III bzw. IV werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Auf diese Weise erhält man ein wasserdispergierbares Pulver, welches die Form III bzw. IV enthält. Durch feines Verteilen der Mischung in 20000 Gewichtsteilen Wasser erhält man eine Spritzbrühe, die 0,1 Gew.-% der Form III bzw. IV enthält.
II. 3 Gewichtsteile der Form III bzw. IV werden mit 97 Gewichtsteilen feinteiligem Kaolin vermischt. Man erhält auf diese Weise ein Stäubemittel, das 3 Gew.-% der Form III bzw. IV enthält.
III. 20 Gewichtsteile der Form III bzw. IV I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenolsulfonsäure-Harnstoff-Formaldehyd-Kondensates und 68 Gewichtsteilen eines paraffinischen Mineralöls innig vermischt. Man erhält eine stabiles nichtwässriges Suspensionskonzentrat der Form III bzw. IV.
IV. 10 Gewichtsteile der Form III bzw. IV wurden in einer Lösung von 17 Gewichtsteilen eines Poly(ethylenglykol)(propylenglykol)blockcopolymeren, 2 Gewichtsteilen eines Phenolsulfonsäure-Formaldehyd-Kondensates und etwa 1 Gewichtsteil sonstigen Hilfsmitteln (Verdicker, Entschäumer) in einem Gemisch aus 7 Gewichtsteilen Propylenglykol und 63 Gewichtsteilen Wasser als Suspensionskonzentrat formuliert.
V. 30,5 Gewichtsteile der Form III bzw. IV wurden in einer Lösung von 1 Gewichtsteil eines Poly(ethylenglykol)(propylenglykol)blockcopolymeren, 1 Gewichtsteil eines Phenolsulfonsäure-Formaldehyd-Kondensates und etwa 1 Gewichtsteil sonstigen Hilfsmitteln (Verdicker, Entschäumer) in einem Gemisch aus 6 Gewichtsteilen Propylenglykol und 61 Gewichtsteilen Wasser als Suspensionskonzentrat formuliert.

Die Applikation der Form III bzw. IV bzw. die der sie enthaltenden herbiziden Mittel erfolgt, sofern die Formulierung nicht bereits gebrauchsfertig ist, in Form wässriger Spritzbrühen. Diese werden durch Verdünnen der vorgenannten, die Form III bzw. IV des Phenyluracils I enthaltenden Formulierungen mit Wasser bereitet. Die Spritzbrühen können auch weitere Bestandteile in gelöster, emulgierter oder suspendierter Form enthalten, beispielsweise Düngemittel, Wirkstoffe anderer herbizider oder wachstumsregulierender Wirkstoffgruppen, weitere Wirkstoffe, z. B. Wirkstoffe zur Bekämpfung von tierischen Schädlingen oder phytopathogenen Pilzen bzw. Bakterien, weiterhin Mineralsalze, welche zur Behebung von Ernährungs- und Spurenelementmängeln eingesetzt werden, sowie nicht-phytotoxische Öle und Ölkonzentrate. In der Regel werden diese Bestandteile vor während oder nach dem Verdünnen der erfindungsgemäßen Formulierungen der Spritzbrühe zugesetzt.

Die Applikation der Form III bzw. IV bzw. die der sie enthaltenden herbiziden Mittel kann im Vorauflauf- oder im Nachauflaufverfahren erfolgen. Sofern das Phenyluracil I für gewisse Kulturpflanzen weniger verträglich ist, können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an der Form III bzw. IV betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte oder zur Selektivitätssteigerung können die Formen III und IV mit zahlreichen Vertretern anderer herbizider oder wachstumsregulierender Wirkstoffgruppen oder mit Safenern gemischt und gemeinsam ausgebracht werden. Die Formen III und IV können beispielsweise in Analogie zu den in WO2003/024221, WO 2004/080183, WO 2006/097509 und WO 2007/042447 beschriebenen Mischungen von Phenyluracilen I mit Herbiziden, Wachstumsregulatoren und/oder Safenern angewendet bzw. appliziert werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht. Geeignete Safener sind beispielsweise (Chinolin-8-oxy)essigsäuren, 1-Phenyl-5-haloalkyl-1H-1,2,4-triazol-3-carbonsäuren, 1-Phenyl-4,5-dihydro-5-alkyl-1 H-pyrazol-3,5-dicarbonsäuren, 4,5-Dihydro-5,5-diaryl-3-isoxazolcarbonsäuren, Dichloroacetamide, alpha-Oximinophenylacetonitrile, Acetophenonoxime, 4,6-Dihalo-2-phenylpyrimidine, N-[[4-(Aminocarbonyl)phenyl]sulfonyl]-2-benzoesäureamide, 1,8-Naphthalsäure-anhydrid, 2-Halo-4-(haloalkyl)-5-thiazolcarbonsäuren, Phosphorthiolate und N-Alkyl-O-phenylcarbamate sowie ihre landwirtschaftlich brauchbaren Salze, und vorausgesetzt sie haben eine Säurefunktion, ihre landwirtschaftlich brauchbaren Derivate, wie Amide, Ester und Thioester.

Außerdem kann es von Nutzen sein, die Form III bzw. IV allein oder in Kombination mit anderen Herbiziden und/oder Safenern auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenetementmängeln eingesetzt werden. Es können auch nicht-phytotoxische Öle und Ölkonzentrate zugesetzt werden.

Die herbizide Wirkung der Formen III und IV ließ sich durch die folgenden Gewächshausversuche zeigen:
Als Kulturgefäße dienten Kunststofftöpfe, die mit Boden (z. B. lehmiger Sand mit etwa 3,0 % Humus) als Substrat gefüllt wurden. Die Samen der Testpflanzen wurden nach Arten getrennt eingesät.

Bei Vorauflaufbehandlung wurden die in Wasser suspendierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 bis 25 °C bzw. 20 bis 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Nach den oben genanten Methoden wurden die erfindungsgemäße Formen III und IV und als Vergleichsverbindung die aus WO 01/83459 bekannte Form I, jeweils formuliert als wässriges Suspensions-Konzentrat (SC; 100 g/l) gegebenenfalls unter Zusatz von 1 I/ha Rustica Öl ® im Gewächshaustest verglichen. Die Suspensionskonzentrate wiesen folgende Zusammensetzung auf:

| | |
|---|---|
| Phenyluracil I | 100 g/L |
| 1,2-Propylenglykol | 70 g/L |
| Dispergiermittel I | 167 g/L |
| Dispergiermittel II | 20 g/L |
| Xanthan-Gum | 3 g/L |
| Biozid | 1,8 g/L |
| Wasser | ad 1 L |

| | |
|---|---|
| Dispergiermittel I: EO/PO-Blockcopolymer Dispergiermittel II: Phenolsulfonsäure-Formaldehyd-Kondensationsprodukt | |

Die in den Gewächshausversuchen verwendeten Pflanzen setzten sich aus folgenden Arten zusammen:

| | |
|---|---|
| Lateinischer Name | Deutscher Name |
| Amaranthus retroflexus | Zurückgekrümmter Fuchsschwanz |
| Ambrosia elatior | Beifußblättrige Ambrosia |
| Capsella bursa-pastoris | Gemeines Hirtentäschelkraut |
| Chenopodium album | Weißer Gänsefuß |
| Euphorbia heterophylla | Wolfsmilch |
| Galium aparine | Klebkraut |
| Glycine max | Sojabohne |
| Helianthus annuus | Sonnenblume |

| Lateinischer Name | Deutscher Name |
|---|---|
| Hordeum vulgare | Sommer- bzw. Wintergerste |
| Kochia scoparia | Besenradmelde |
| Matricaria inodora | Geruchlose Kamille |
| Mercurialis annua | Einjähriges Bingelkraut |
| Polygonum convolvulus | Windenknöterich |
| Salsola kali ssp. ruthenica | Russisches Salzkraut |
| Secale cereale | Winterroggen |
| Sinapis arvensis | Ackersenf |
| Sonctius arvensis | Ackergänsediestel |
| Stellaria media | Vogelsternmiere |
| Thlaspi arvense | Ackerhellerkraut |
| Triticum aestivum | Sommerweizen |
| Veronica hederaefolia | Efeublättriger Ehrenpreis |
| Veronica persicaria | Persischer Ehrenpreis |
| Viola arvensis | Acherstiefmütterchen |

**Tabelle 4 Vergleich der herbiziden Wirksamkeit der Form III mit der aus WO 01/83459 bekannten Form I im Vorauflauf (Gewächshaus)**

| | | | Wirkstoff | |
|---|---|---|---|---|
| | Testpflanzen | Aufwandmenge (g/ha a. S.) | Form III | Modifikation I |
| | | | Schaden[%] | |
| Nutzpflanze: | | | | |
| | Glycine max | 25 | 30 | 70 |
| | | 12,5 | 20 | 30 |
| | | | | |
| Schadpflanze: | | | | |
| | Stellaria media | 12,5 | 70 | 65 |
| | Ambrosia elatior | 12,5 | 80 | 60 |
| | Helianthus annuus | 12,5 | 75 | 70 |
| | Euphorbia heterophylla | 12,5 | 100 | 95 |
| | | 6,25 | 90 | 40 |
| | Mercurialis annua | 6,25 | 65 | 40 |

**Tabelle 5 Vergleich der herbiziden Wirksamkeit der Form III mit der aus WO 01/83459 bekannten Form I im Nachauflauf unter Zusatz von 1 I/ha Rustica Öl® (Gewächshaus)**

| | | | Wirkstoff | |
|---|---|---|---|---|
| | Testpflanzen | Aufwandmenge (g/ha a. S.) | Form III | Form I |
| | | | Schaden [%] | |
| Nutzpflanze: | | | | |
| | Hordeum vulgare (Sommer-) | 20 | 5 | 15 |
| | Hordeum vulgare (Winter-) | 20 | 5 | 20 |
| | | 15 | 5 | 15 |
| | Secale cereale | 20 | 5 | 15 |
| | | 15 | 0 | 10 |
| | | 10 | 0 | 10 |
| | Triticum aestivum | 20 | 10 | 15 |
| | | | | |
| Schadpflanze: | | | | |
| | Chenopodium album | 15 | 80 | 70 |
| | Galium aparine | 15 | 100 | 75 |
| | Matricaria inodora | 5 | 100 | 65 |
| | Polygonum convolvulus | 15 | 100 | 70 |
| | Veronica hederaefolia | 15 | 70 | 50 |
| | Veronica persicaria | 15 | 100 | 70 |
| | Viola arvensis | 5 | 100 | 40 |

**Tabelle 6 Vergleich der herbiziden Wirksamkeit der Form III mit der aus WO 01/83459 bekannten Form I im Nachauflauf (Gewächshaus)**

| | | | Wirkstoff | |
|---|---|---|---|---|
| | Testpflanzen | Aufwandmenge | Form III | Form I |
| | | (g/ha a. S.) | Schaden [%] | |
| Nutzpflanze: | | | | |
| | Hordeum vulgare (Winter-) | 15 | 0 | 5 |
| | Secale cereale | 20 | 5 | 15 |
| | Triticum aestivum | 20 | 5 | 10 |
| | | | | |
| Schadpflanze: | | | | |
| | Amaranthus retroflexus | 10 | 100 | 40 |
| | Capsella bursa-pastoris | 15 | 70 | 20 |
| | Kochia scoparia | 20 | 100 | 45 |
| | Matricaria inodora | 15 | 100 | 65 |
| | Salsola kali ssp. ruthenica | 20 | 100 | 80 |
| | Sinapis arvensis | 10 | 60 | 50 |
| | Sonchus arvensis | 15 | 100 | 60 |
| | Thlaspi arvense | 10 | 100 | 30 |
| | Veronica persicaria | 15 | 100 | 40 |
| | Viola arvensis | 15 | 100 | 40 |

Die Testergebnisse zeigen deutlich, dass die erfindungsgemäße Form III gegenüber der bekannten Form I eine verbesserte Toleranz durch die Nutzpflanze bei gleichzeitiger deutlich verbesserter herbizider Wirksamkeit aufweist.

## Patentansprüche

1. Kristalline Hydrate des 2-Chlor-5-(3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid.

2. Hydrate nach Anspruch 1, enthaltend 0,8 bis 1,2 Mol Wasser, bezogen auf 1 Mol 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid.

3. Hydrate nach Anspruch 1 oder 2 mit einem Schmelzpeak im Bereich von 100 bis 140 °C.

4. Hydrat nach einem der vorhergehenden Ansprüche mit einem Gehalt an 2-Chlor-5-[3,6-dihydro-3-methyl-2,6-dioxo-4-(trifluormethyl)-1-(2H)-pyrimidinyl]-4-fluor-N-[[methyl-(1-methylethyl)aminolsulfonyl]benzamid von wenigstens 94 Gew.-%, bezogen auf die Gesamtmenge der im Hydrat enthaltenen organischen Bestandteile.

5. Hydrat (a) nach einem der vorhergehenden Ansprüche, das in einem Röntgenpulverdiffraktogramm bei 25 °C und Cu-K_{α}-Strahlung zumindest einen Reflex bei dem 2θ-Wert 11,6 ± 0,2° zeigt.

6. Hydrat nach Anspruch 5, das zusätzlich wenigstens drei der folgenden, als 2θ-Werte angegebenen Reflexe zeigt: 5,1 ± 0,2°, 10,1 ± 0,2°, 10,8 ± 0,2°, 13,9 ± 0,2°, 15,1 ± 0,2°, 16,1 ± 0,2°, 17,9 ± 0,2°, 20,2 ± 0,2°, 24,5 ± 0,2°.

7. Hydrat (b) nach einem der Ansprüche 1 bis 4, das in einem Röntgenpulverdiffraktogramm bei 25 °C und Cu-K_{α}-Strahlung zumindest einen Reflex bei dem 2θ-Wert 12,1 ± 0,2° zeigt.

8. Hydrat nach Anspruch 7, das zusätzlich wenigstens drei der folgenden, als 2θ-Werte angegebenen Reflexe zeigt: 5,2 ± 0,2°, 10,2 ± 0,2°, 10,9 ± 0,2°, 14,0 ± 0,2°, 14,6 ± 0,2°,15,3 ± 0,2°, 19,2 ± 0,2°, 19,9 ± 0,2°, 20,5 ± 0,2°, 24,7 ± 0,2°, 26,7 ± 0,2°, 27,8± 0,2°.

9. 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid, bestehend zu wenigstens 90 %, bezogen auf die Gesamtmenge an 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid, aus einem Hydrat gemäß einem der vorhergehenden Ansprüche.

10. Verfahren zur Herstellung eines Hydrats gemäß einem der Ansprüche 1 bis 8, umfassend die Kristallisation aus einer Lösung des 2-Chlor-5-[3,6-dihydro-3-rnethyl-2,6-dioxo-4-(trifluormethyl)-1-(2H)-pyrimidinyl]-4-fluor-N-[[methyl-(1-methylethyl)amino]sulfonyl]benzamids in einem organischen Lösungsmittel in Gegenwart von Wasser.

11. Verfahren zur Herstellung eines Hydrats gemäß einem der Ansprüche 1 bis 8, umfassend das Suspendieren von amorphem 2-Chlor-5-[3,6-dihydro-3-methyl-2,6-dioxo-4-(trifluormethyl)-1-(2H)-pyrimidinyl]-4-fluor-N-[[methyl-(1-methylethyl)-amino]sulfonyl]benzamid in Wasser oder einem wasserhaltigen organischen Lösungsmittel.

12. Pflanzenschutzmittel, enthaltend ein Hydrat gemäß einem der Ansprüche 1 bis 8 und für die Formulierung von Pflanzenschutzmitteln übliche Hilfsmittel.

13. Verfahren zur Bekämpfung von unerwünschtem Pflanzenwuchs, **dadurch gekennzeichnet, dass** man ein Hydrat des 2-Chlor-5-[3,6-dihydro-3-methyl-2,6-dioxo-4-(trifluormethyl)-1-(2H)-pyrimidinyl]-4-fluor-N-[[methyl-(1-methy)ethyl)-amino]sulfonyl]benzamids nach einem der Ansprüche 1 bis 8 auf Pflanzen, deren Lebensraum und/oder auf Samen einwirken lässt.

## Claims

1. A crystalline hydrate of 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide.

2. The hydrate as claimed in claim 1, comprising from 0.8 to 1.2 mol of water per mole of 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide.

3. The hydrate as claimed in claim 1 or 2 having a melting peak in the range of from 100 to 140°C.

4. The hydrate as claimed in any of the preceding claims having a 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]-sulfonyl]benzamide content of at least 94% by weight, based on the total amount of the organic components contained in the hydrate.

5. The hydrate (a) as claimed in any of the preceding claims which, in an X-ray powder diffractogram, at 25°C and with Cu-K_{α} radiation, shows at least a reflex at 2θ 11.6 ± 0.2°.

6. The hydrate as claimed in claim 5 which additionally shows at least three of the reflexes below, stated as 2θ values: 5.1 ± 0.2°, 10.1 ± 0.2°, 10.8 ± 0.2°, 13.9 ± 0.2°, 15.1 ± 0.2°, 16.1 ± 0.2°, 17.9 ± 0.2°, 20.2 ± 0.2°, 24.5 ± 0.2°.

7. The hydrate (b) according to any of claims 1 to 4 which, in an X-ray powder diffractogram, at 25°C and with Cu-K_{α} radiation, shows at least a reflex at 2θ 12.1 ± 0.2°.

8. The hydrate as claimed in claim 7 which additionally shows at least three of the reflexes below, stated as 2θ values: 5.2 ± 0.2°, 10.2 ± 0.2°, 10.9 ± 0.2°, 14.0 ± 0.2°, 14.6 ± 0,2°, 15.3 ± 0.2°, 19.2 ± 0.2°, 19.9 ± 0.2°, 20.5 ± 0,2°, 24.7 ± 0.2°, 26.7 ± 0.2°, 27.8 ± 0.2°.

9. 2-Chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide, consisting of at least 90%, based on the total amount of 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]-sulfonyl]benzamide, of a hydrate as claimed in any of the preceding claims.

10. A process for preparing a hydrate as claimed in any of claims 1 to 8, comprising the crystallization from a solution of 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide in an organic solvent in the presence of water.

11. A process for preparing a hydrate as claimed in any of claims 1 to 8, comprising the suspension of amorphous 2-chloro-5-{3,6-dihydro-3-methyl-2,6-dioxo-4-(trifluoromethyl)-1-(2H)-pyrimidinyl]-4-fluoro-N-[[methyl-(1-methylethyl)-amino]sulfonyl]-benzamide in water or an aqueous organic solvent.

12. A crop protection composition, comprising a hydrate as claimed in any of claims 1 to 8 and auxiliaries customary for formulating crop protection agents.

13. A method for controlling unwanted vegetation, which comprises allowing a hydrate of 2-chloro-5-{3,6-dihydro-3-methyl-2,6-dioxo-4-(trifluoromethyl)-1-(2H)-pyrimidinyl]-4-fluoro-N-[[methyl-(1-methylethyl)-amino]sulfonyl]benzamide as claimed in any of claims 1 to 8 to act on plants, their habitat and/or on seed.

## Revendications

1. Hydrates cristallins du 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]benzamide.

2. Hydrates selon la revendication 1, contenant 0,8 à 1,2 mole d'eau par rapport à 1 mole de 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]benzamide.

3. Hydrates selon la revendication 1 ou 2 présentant un pic de fusion dans la plage de 100 à 140°C.

4. Hydrate selon l'une quelconque des revendications précédentes, présentant une teneur en 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]benzamide d'au moins 94% % en poids, par rapport à la quantité totale des constituants organiques contenus dans l'hydrate.

5. Hydrate (a) selon l'une quelconque des revendications précédentes, qui présente dans le diffractogramme de rayons X sur des poudres à 25°C et avec un rayonnement Cu-K_{α} au moins une réflexion à la valeur 2θ de 11,6 ± 0,2°.

6. Hydrate selon la revendication 5, qui présente en outre au moins trois des réflexions suivantes, indiquées comme valeurs 2θ: 5,1 ± 0,2°, 10,1 ± 0,2°, 10,8 ± 0,2°, 13,9 ± 0,2°, 15,1 ± 0,2°, 16,1 ± 0,2°, 17,9 ± 0,2°, 20,2 ± 0,2°, 24,5 ± 0,2°.

7. Hydrate (b) selon l'une quelconque des revendications 1 à 4, qui présente dans le diffractogramme de rayons X sur des poudres à 25°C et avec un rayonnement Cu-K_{α} au moins une réflexion à la valeur 2α de 12,1 ± 0,2°.

8. Hydrate selon la revendication 7, qui présente en outre au moins trois des réflexions suivantes, indiquées comme valeurs 2θ : 5,2 ± 0,2°, 10,2 ± 0,2°, 10,9 ± 0,2°, 14,0 ± 0,2°, 14,6 ± 0,2°, 15,3 ± 0,2°, 19,2 ± 0,2°, 19,9 ± 0,2°, 20,5 ± 0,2°, 24,7 ± 0,2°, 26,7 ± 0,2°, 27,8 ± 0,2°.

9. 2-chloro-5[3,6-dihydro-3-méthyl-2,5-dioxo-4-(trifluorométhyl)-1-(2H)-pyrimidinyl]-4-fluoro-N-[[méthy1-(1-méthyléthyl)amino]sulfonyl]benzamide, constitué à raison d'au moins 90%, par rapport à la quantité totale de 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]benzamide, d'un hydrate selon l'une quelconque des revendications précédentes.

10. Procédé pour la préparation d'un hydrate selon l'une quelconque des revendications 1 à 8, comprenant la cristallisation à partir d'une solution du 2-chloro-5-[3,6-dihydro-3-méthyl-2,6-dioxo-4-(trifluorométhyl)-1-(2H)-pyrimidinyl1-4-fluoro-N-[[méthyl-(1-méthyléthyl)amino]sulfonyl]benzamide dans un solvant organique en présence d'eau.

11. Procédé pour la préparation d'un hydrate selon l'une quelconque des revendications 1 à 8, comprenant la mise en suspension de 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)-amino]sulfonyl]benzamide amorphe dans l'eau ou un solvant organique contenant de l'eau.

12. Agent de phytoprotection, contenant un hydrate selon l'une quelconque des revendications 1 à 8 et des adjuvants usuels pour la formulation d'agents de phytoprotection.

13. Procédé pour lutter contre une croissance non souhaitée de plantes, **caractérisé en ce qu'**on laisse agir un hydrate du 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)-amino]sulfonyl]benzamide selon l'une quelconque des revendications 1 à 8 sur des plantes, leur espace de vie et/ou des graines.
